# EUROPEAN PATENT APPLICATION

(11) **EP 2 382 988 A1**
(43) Date of publication of application: **02.11.2011**
(21) Application number: 11158008.0
(22) Date of filing: 29.03.2007
(51) Int. Cl.: A61K 39/21

(54) **Combined mucosal and parenteral immunization against HIV**

(30) Priority: 31.03.2006 US 788563 P; 26.08.2006 US 840178 P
(62) Divisional of application: 07773929.0
(71) Applicant: Novartis AG, 4056 Basel (CH)
(72) Inventor: Rappuoli, Rino, Emeryville, CA 94662-8097 (US); Lewis, David J. M., Emeryville, CA 94662-8097 (US); Barnett, Susan W., Emeryville, CA 94662-8097 (US)
(74) Representative: Marshall, Cameron John

(57) **Abstract**

HIV antigens are mucosally administered in one or more priming immunization(s), and then HIV antigens are parenterally administered in one or more boosting immunization(s). Thus the invention provides a method for raising an immune response in a patient, comprising: (i) administering a HIV antigen to the patient via a mucosal route; and then (ii) administering a HIV antigen to the patient via a parenteral route. The antigens will typically be adjuvanted. Preferred mucosal immunizations are via the intranasal route using a detoxified mutant of E.coli heat labile toxin as the adjuvant. Preferred parenteral immunizations are via the intramuscular route using an oil-in-water emulsion adjuvant.

## Description

### RELATED APPLICATIONS

This application claims the benefit of U.S. Provisional Application No. 60/788,563, filed March 31, 2006, and U.S. Provisional Application No. 60/840,178, filed August 26, 2006. Both of these applications are incorporated herein by reference in their entirety.

### TECHNICAL FIELD

This invention is in the field of human immunodeficiency virus (HIV) and, in particular, compositions and methods for use in HIV-1 immunization.

### BACKGROUND OF THE INVENTION

Vaccines for immunizing patients against HIV infection have been under development for two decades, but with limited success. Many approaches to immunization have focused on the HIV envelope glycoprotein, although there has also been interest in other antigens such as tat or gag.

Reference 1 discloses compositions comprising a HIV antigen, such as an envelope antigen, and a mucosal adjuvant, such as a detoxified mutant of *E.coli* heat labile toxin (LT). The compositions were administered to rhesus macaques intranasally and an antibody response was obtained.

Reference 2 discloses HIV immunization regimens involving parenteral priming and mucosal boosting.

It is an object to provide further and improved ways of mucosally immunizing against HIV.

### SUMMARY OF THE INVENTION

The present invention relates to methods method for raising an immune response in a patient. In one embodiment, the method for raising an immune response in a patient comprises: (i) administering a HIV-1 envelope antigen to the patient via a mucosal route; and then (ii) administering a HIV-1 envelope antigen to the patient via a parenteral route. In another embodiment, the method for raising an immune response in a patient comprises administering a HIV-1 envelope antigen to the patient via a parenteral route, wherein the patient has previously received a HIV-1 envelope antigen via a mucosal route.

The present invention also relates to uses of an HIV-1 envelope antigen in the manufacture of a medicament for administration to a patient by a method comprising: (i) administering the HIV-1 envelope antigen to the patient via a mucosal route; and then (ii) administering a HIV-1 envelope antigen to the patient via a parenteral route.

The invention further relates to uses of an HIV-1 envelope antigen in the manufacture of a medicament for administration to a patient who has previously received a HIV-1 envelope antigen via a mucosal route, wherein the medicament is for administration to the patient via a parenteral route.

The present invention also relates to kits comprising (i) an HIV-1 envelope antigen for administration to a patient via a mucosal route; and (ii) an HIV-1 envelope antigen for administration to a patient via a parenteral route.

The invention further relates to an HIV-1 envelope antigen in combination with a mucosal adjuvant, and a HIV-1 envelope antigen in combination with a parenteral adjuvant, for simultaneous separate or sequential administration.

The present invention also relates to immunogenic compositions for intranasal administration. In one embodiment, the immunogenic composition for intranasal administration comprises an HIV-1 envelope antigen in combination with a detoxified mutant of *E.coli* heat labile toxin ('LT'), wherein a unit dose of the composition includes between 10µg and 1mg of Env antigen and between 10µg and 50µg of LT. In other embodiments, the immunogenic composition for intranasal administration comprises an HIV-1 envelope antigen in combination with a detoxified mutant of *E.coli* heat labile toxin ('LT'), wherein the weight ratio of Env antigen to LT is between 2:1 and 4:1.

The present invention further relates to immunogenic compositions for intramuscular administration, comprising an HIV-1 envelope antigen in combination with an oil-in-water emulsion adjuvant, wherein a unit dose of the composition includes between 10µg and 1mg of envelope antigen.

In some embodiments, the HIV-1 envelope antigens of the methods, uses and kits of the invention are adjuvanted. In other embodiments, mucosal immunizations are via the intranasal route. In still other embodiments, parenteral immunizations are via the intramuscular route.

In particular embodiments, mucosal immunizations include a detoxified mutant of *E.coli* heat labile toxin as adjuvant. In other embodiments, parenteral immunizations include an oil-in-water emulsion as adjuvant.

More than one mucosal immunization can be administered in the methods, uses and kits of the invention. In some embodiments, mucosal immunization is intranasal. In certain embodiments, the mucosal immunization has three intranasal doses of a composition with between 10µg and 50µg of LT-K63 adjuvant per dose.

More than one parenteral immunization can be administered in the methods, uses and kits of the invention. In some embodiments, parenteral immunization is intramuscular. In certain embodiments, the parenteral immunization has two intramuscular doses of a composition with a squalene-in-water emulsion.

In some embodiments, each immunization uses the same HIV-1 envelope antigen. In other embodiments, each immunization uses a different HIV-1 envelope antigen. In a particular embodiment, the envelope antigen is o-gp140Δ V2_{SF162}.

The methods, uses and kits of the invention can be used (employed) in cases where the patient is not infected with HIV-1. The methods, uses and kits of the invention can also be employed (used) where the patient is infected with HIV-1. In particular embodiments, the patient is a human aged between 12 and 55 years old.

### BRIEF DESCRIPTION OF THE DRAWINGS

FIG. 1 shows IgG responses in mice after three intranasal doses.
FIG. 2 shows the same responses after the mice received two further intramuscular doses.
FIG. 3 shows IgA responses for the same mice as in Figure 2.

### DETAILED DESCRIPTION OF THE INVENTION

Reference 2 discloses HIV immunization regimens involving parenteral priming and mucosal boosting. In contrast, in the present invention administers HIV-1 envelope antigen is mucosally in one or more priming immunization(s), and then parenterally in one or more boosting immunization(s). The initial mucosal immunization primes both the systemic immune compartment and, via the common mucosal immune system, seed cells in diverse and distant mucosal sites such as the rectum, vagina and cervix. Both T cell and antibody immunity can be elicited at these mucosal sites. The primary antibody and CD4+ T cell responses induced by the mucosal immunizations can be amplified by the parenteral booster immunization. Compared to the mucosal administration used in reference 1, the data herein show that addition of parenteral boosting increases both the serum and mucosal antibody responses.

Thus the invention provides a method for raising an immune response in a patient, comprising:
(i) administering a HIV-1 envelope antigen to the patient via a mucosal route; and then (ii) administering a HIV-1 envelope antigen to the patient via a parenteral route.

The invention also provides a method for raising an immune response in a patient, comprising: administering a HIV-1 envelope antigen to the patient via a parenteral route, wherein the patient has previously received a HIV-1 envelope antigen via a mucosal route.

The invention provides the use of a HIV-1 envelope antigen in the manufacture of a medicament for administration to a patient by a method comprising: (i) administering the HIV-1 envelope antigen to the patient via a mucosal route; and then (ii) administering a HIV-1 envelope antigen to the patient via a parenteral route.

The invention provides the use of a HIV-1 envelope antigen in the manufacture of a medicament for administration to a patient who has previously received a HIV-1 envelope antigen via a mucosal route, wherein the medicament is for administration to the patient via a parenteral route.

The invention also provides a kit comprising (i) a HIV-1 envelope antigen for administration to a patient via a mucosal route; and (ii) a HIV-1 envelope antigen for administration to a patient via a parenteral route. The kit may be combined (e.g. in the same box) with a leaflet including details of the composition e.g. instructions for administration, details of the antigens within the composition, *etc.* The instructions may also contain warnings e.g. to keep a solution of adrenaline readily available in case of anaphylactic reaction following vaccination, *etc.*

The antigens will typically be adjuvanted.

Thus the invention provides (i) a HIV-1 envelope antigen in combination with a mucosal adjuvant; and (ii) a HIV-1 envelope antigen in combination with a parenteral adjuvant, for simultaneous separate or sequential administration.

Preferred mucosal immunizations are via the intranasal route using a detoxified mutant of *E.coli* heat labile toxin as the adjuvant. Preferred parenteral immunizations are via the intramuscular route using an adjuvant capable of inducing (eliciting) a Thl-type immune response in a subject. In a particular embodiment, the adjuvant is an oil-in-water emulsion adjuvant.

The invention also provides an immunogenic composition for intranasal administration, comprising a HIV envelope antigen in combination with a detoxified mutant of *E.coli* heat-labile toxin ('LT'). A unit dose of the composition may include between 10µg and 1mg of envelope antigen e.g. about 100µg. A unit dose of the composition may include between 10µg and 50µg of LT e.g. about 30µg. A unit dose for intranasal administration typically has a volume of about 300µ1. This composition can be used in the methods, uses and kits of the invention.

The invention also provides an immunogenic composition for intranasal administration, comprising a HIV-1 envelope antigen in combination with a detoxified mutant of *E.co*/*i* heat-labile toxin ('LT'), wherein the weight ratio of envelope antigen to LT is between 2:1 and 4:1 e.g. about 10:3. This composition can be used in the methods, uses and kits of the invention.

The invention also provides an immunogenic composition for intramuscular administration, comprising a HIV-1 envelope antigen in combination with an adjuvant capable of inducing (eliciting) a Th1-type immune response in a subject. In a particular embodiment, the adjuvant is an oil-in-water emulsion adjuvant. A unit dose of the composition may include between 10µg and 1mg of envelope antigen e.g. about 100µg. This composition can be used in the methods, uses and kits of the invention.

### The HIV-1 envelope antigen(s)

Where more than one mucosal immunization is administered, the HIV envelope antigen(s) in each immunization may be the same as or different from each other. Similarly, where more than one parenteral immunization is administered, the HIV envelope antigen(s) in each immunization may be the same as or different from each other. Moreover, the HIV envelope antigen(s) that is/are mucosally administered may be the same as or different from the HIV envelope antigen(s) that is/are parenterally administered. In typical embodiments, however, the mucosal immunization(s) and the parenteral administration(s) will use the same HIV envelope antigen(s).

Where different HIV envelope antigens are used, the antigens may be from the same group e.g. both are from group M, group N or group O. Within group M, the envelope antigens may be from the same subtype (or clade) *e.g.* from subtype A, B, C, D, F, G, H, J or K. It is also possible to use envelope antigens from a CRF (circulating recombinant form) subtype, such as a A/B or A/E CRF. Where a subtype includes sub-subtypes then the envelope antigens may be from the same sub-subtype. In other embodiments, envelope antigens are from different groups, subtypes and/or sub-subtypes. HIV-1 nomenclature is discussed in more detail in reference 3.

The HIV-1 envelope protein (Env) is initially expressed as a long precursor protein ('gp160') that is subsequently cleaved to give an exterior membrane glycoprotein ('gp120') and a transmembrane glycoprotein ('gp41'). The invention can use various forms of Env protein. For example, the invention may use a full-length gp160 polypeptide, a gp120 polypeptide, a gp160 or gp120 polypeptide with one or more deletions, a fusion protein including a gp120 or gp160 polypeptide, etc. Rather than being a full-length gp160 precursor, however, the invention will typically use a shortened protein.

The amino acid sequence of the full-length HIV-1 Env precursor from the REFSEQ database (GI:9629363) is a 856mer shown below (SEQ ID NO: 1 herein):

MRVKEKYQHLWRWGWRWGTMLLGMLMICSATEKLWVTVYYGVPVWKEATTTLFCASDAKAYDTEVHNVWATHA
CVPTDPNPQEVVLVNVTENFNMWKNDMVEQMHEDIISLWDQSLKPCVKLTPLCVSLKCTDLKNDTNTNSSSGR
MIMEKGEIKNCSFNISTSIRGKVQKEYAFFYKLDIIPIDNDTTSYKLTSCNTSVITQACPKVSFEPIPIHYCA
PAGFAILKCNNKTFNGTGPCTNVSTVQCTHGIRPVVSTQLLLNGSLAEEEVVIRSVNFTDNAKTIIVQLNTSV
EINCTRPNNNTRKRIRIQRGPGRAFVTIGKIGNMRQAHCNISRAKWNNTLKQIASKLREQFGNNKTIIFKQSS
GGDPEIVTHSFNCGGEFFYCNSTQLFNSTWFNSTWSTEGSNNTEGSDTITLPCRIKQIINMWQKVGKAMYAPP
ISGQIRCSSNITGLLLTRDGGNSNNESEIFRPGGGDMRDNWRSELYKYKVVKIEPLGVAPTKAKRRVVQREKR
AVGIGALFLGFLGAAGSTMGAASMTLTVQARQLLSGIVQQQNNLLRAIEAQQHLLQLTVWGIKQLQARILAVE
RYLKDQQLLGIWGCSGKLICTTAVPWNASWSNKSLEQIWNHTTWMEWDREINNYTSLIHSLIEESQNQQEKNE
QELLELDKWASLWNWFNITNWLWYIKLFIMIVGGLVGLRIVFAVLSIVNRVRQGYSPLSFQTHLPTPRGPDRP
EGIEEEGGERDRDRSIRLVNGSLALIWDDLRSLCLFSYHRLRDLLLIVTRIVELLGRRGWEALKYWWNLLQYW
SQELKNSAVSLLNATAIAVAEGTDRVIEVVQGACRAIRHIPRRIRQGLERILL

The wild-type HIV-1 precursor protein is cleaved to give the surface glycoprotein gp120 (e.g. amino acids 29-511 of SEQ ID NO: 1; SEQ ID NO: 2 herein) and the transmembrane domain gp41 (e.g. amino acids 512-856 of SEQ ID NO: 1; SEQ ID NO: 3 herein):

MRVKEKYQHLWRWGWRWGTMLLGMLMIC/SATEKLWVTVYYGVPVWKEATTTLFCASDAKAYDTEVHNVWATH
ACVPTDPNPQEVVLVNVTENFNMWKNDMVEQMHEDIISLWDQSLKPCVKLTPLCVSLKCTDLKNDTNTNSSSG
RMIMEKGEIKNCSFNISTSIRGKVQKEYAFFYKLDIIPIDNDTTSYKLTSCNTSVITQACPKVSFEPIPIHYC
APAGFAILKCNNKTFNGTGPCTNVSTVQCTHGIRPVVSTQLLLNGSLAEEEVVIRSVNFTDNAKTIIVQLNTS
VEINCTRPNNNTRKRIRIQRGPGRAFVTIGKIGNMRQAHCNISRAKWNNTLKQIASKLREQFGNNKTIIFKQS
SGGDPEIVTHSFNCGGEFFYCNSTQLFNSTWFNSTWSTEGSNNTEGSDTITLPCRIKQIINMWQKVGKAMYAP
PISGQIRCSSNITGLLLTRDGGNSNNESEIFRPGGGDMRDNWRSELYKYKVVKIEPLGVAPTKAKRRVVQREK
R/AVGIGALFLGFLGAAGSTMGAASMTLTVQARQLLSGIVQQQNNLLRAIEAQQHLLQLTVWGIKQLQARILA
VERYLKDQQLLGIWGCSGKLICTTAVPWNASWSNKSLEQIWNHTTWMEWDREINNYTSLIHSLIEESQNQQEK
NEQELLELDKWASLWNWFNITNWLWYIKLFIMIVGGLVGLRIVFAVLSIVNRVRQGYSPLSFQTHLPTPRGPD
RPEGIEEEGGERDRDRSIRLVNGSLALIWDDLRSLCLFSYHRLRDLLLIVTRIVELLGRRGWEALKYWWNLLQ
YWSQELKNSAVSLLNATAIAVAEGTDRVIEVVQGACRAIRHIPRRIRQGLERILL

The hypervariable regions within the gp120 region are located as follows, numbered according to SEQ ID NO: 1: V1 = 131-157; V2 = 157-196; V3 = 296-331; V4 = 385-418; and V5 = 461-471. Within the overall C1-V1-V2-C2-V3-C3-V4-C4-V5-C5 arrangement of gp120, therefore, the subdomains are as follows (numbered according to SEQ ID NO: 2): 1-102; 103-129; 129-168; 169-267; 268-303; 304-356; 357-390; 391-432; 433-443; and 444-483. Residues that have been identified as important for CD4 binding include (numbered according to SEQ ID NO: 1) Asp-368, Glu-370, Trp-427, Val-430 and Pro-438, and the immunodominant region is residues 588-607. These features can be identified in other HIV-1 Env sequences by performing a suitable sequence alignment. Pre-aligned sequences from numerous strains, annotated with these features, can also be found in the *Los Alamos HIV Sequence Compendia* [4].

Other Env sequences that can be used include those disclosed in references 5 to 9

As mentioned above, the invention will typically use a shortened Env polypeptide. The shortening will involve the removal of one of more amino acids from the full-length sequence e.g. truncation at the C-terminus and/or N-terminus, deletion of internal residues, removal of subdomains [10], and combinations of these approaches.

For instance, it is known to make a soluble form of the Env precursor by removing its transmembrane domain and cytoplasmic tail. This polypeptide, which includes the gp120 sequence and the ectodomain of gp41, is known as 'gp140' [11], and has been reported to be a better immunogen than gp120 [12]. Thus the precursor is truncated at its C-terminus e.g. after Lys-665 of SEQ ID NO:1, giving a mature gp140 sequence of a 637mer (SEQ ID NO:4 herein) having amino acids Ser-29 to Lys-665 of SEQ ID NO: 1. Thus the Env polypeptide of the invention may include a portion of gp41 but not include its transmembrane domain.

It is also known to make deletions within the V2 loop of the Env precursor, to give 'ΔV2' mutants. For instance, one or more amino acids within the 40-mer V2 loop can be deleted (*e.g.* at least 2, 3, 4, 5, 6, 7, 8, 9, 10, 12, 14, 16, 18, 20, 22, 24, 26, 28, 30, 32, 34, 36, 38 or more amino acids). Deletions within the V2 loop have been reported to improve immunogenicity of Env polypeptides [13,14]. Env polypeptides with deletions and/or substitutions in the V2 loop are preferred with the present invention, as these have been found to be particularly useful in forming Env/Tat complexes. In particular, Env/Tat complexes are not seen with monomeric gp120 unless its V2 loop is mutated. Amino acids deleted from the V2 loop may be substituted with other amino acids e.g. it is known to replace the central portion of the V2 loop with a Gly-Ala-Gly tripeptide. For example, a ΔV2 mutant may have the following sequence (SEQ ID NO: 5): 
SATEKLWVTVYYGVPVWKEATTTLFCASDAKAYDTEVHNVWATHACVPTDPNPQEVVLVNVTENFNMWKNDMV
EQMHEDIISLWDQSLKPCVKLTPLCVSLKCTDLKNDTNTNSSSGRMIMEKGEIKNCXCNTSVITQACPKVSFE
PIPIHYCAPAGFAILKCNNKTFNGTGPCTNVSTVQCTHGIRPVVSTQLLLNGSLAEEEVVIRSVNFTDNAKTI
IVQLNTSVEINCTRPNNNTRKRIRIQRGPGRAFVTIGKIGNMRQAHCNISRAKWNNTLKQIASKLREQFGNNK
TIIFKQSSGGDPEIVTHSFNCGGEFFYCNSTQLFNSTWFNSTWSTEGSNNTEGSDTITLPCRIKQIINMWQKV
GKAMYAPPISGQIRCSSNITGLLLTRDGGNSNNESEIFRPGGGDMRDNWRSELYKYKVVKIEPLGVAPTKAKR
RVVQREKR
 where the 'X' at position 130 represents a mutant V2 loop e.g. with between 4 and 15 amino acids.

A particularly preferred Env polypeptide for use with the invention is a gp140 protein with a ΔV2 mutation from HIV-1 strain SF162. In its mature form, after cleavage of a signal sequence and secretion (see Figure 24 of reference 5), this polypeptide has the following amino acid sequence (SEQ ID NO: 6; 'gp140ΔV2_{SF162}'):

SAVEKLWVTVYYGVPVWKEATTTLFCASDAKAYDTEVHNVWATHACVPTDPNPQEIVLENVTENFNMWKNNMV
EQMHEDIISLWDQSLKPCVKLTPLCVTLHCTNLKNATNTKSSNWKEMDRGEIKNCSFKVGAGKLINCNTSVIT
QACPKVSFEPIPIHYCAPAGFAILKCNDKKFNGSGPCTNVSTVQCTHGIRPVVSTQLLLNGSLAEEGVVIRSE
NFTDNAKTIIVQLKESVEINCTRPNNNTRKSITIGPGRAFYATGDIIGDIRQAHCNISGEKWNNTLKQIVTKL
QAQFGNKTIVFKQSSGGDPEIVMHSFNCGGEFFYCNSTQLFNSTWNNTIGPNNTNGTITLPCRIKQIINRWQE
VGKAMYAPPIRGQIRCSSNITGLLLTRDGGKEISNTTEIFRPGGGDMRDNWRSELYKYKVVKIEPLGVAPTKA
ISSVVQSEKSAVTLGAMFLGFLGAAGSTMGARSLTLTVQARQLLSGIVQQQNNLLRAIEAQQHLLQLTVWGIK
QLQARVLAVERYLKDQQLLGIWGCSGKLICTTAVPWNASWSNKSLDQIWNNMTWMEWEREIDNYTNLIYTLIE
ESQNQQEKNEQELLELDKWASLWNWFDISKWLWYI

As the HIV genome is in a state of constant flux, and contains several domains that exhibit relatively high degrees of variability between isolates, the invention is not limited to the use of Env polypeptides having the exact sequence of a known HIV polypeptide. Thus the Env polypeptide used according to the invention may be selected from:
(i) a polypeptide comprising an amino acid sequence selected from SEQ ID NOs: 1, 2, 4, 5 and 6;
(ii) a polypeptide comprising an amino acid sequence that has sequence identity to an amino acid sequence selected from SEQ ID NOs: 1, 2, 4, 5 and 6;
(iii) a polypeptide comprising an amino acid sequence that, compared to an amino acid sequence selected from SEQ ID NOs: 1, 2, 4, 5 and 6, has one or more (e.g. 1, 2, 3, 4, 5, 6, 7, 8, 9, 10, etc.) substitutions and/or deletions and/or insertions;
(iv) a polypeptide comprising an amino acid sequence comprising a fragment of at least n consecutive amino acids from an amino acid sequence selected from SEQ ID NOs: 1, 2, 4, 5 and 6, where n is 7 or more; or
(v) a polypeptide comprising a sequence of *p* amino acids that, when aligned with an amino acid sequence selected from SEQ ID NOs: 1, 2, 4, 5 and 6 using a pairwise alignment algorithm, has at least *x·y* identical aligned monomers in each window of x amino acids moving from N-terminus to C-terminus, where: p>x; there are *p-x+1* windows; x is selected from 20, 25, 30, 35, 40, 45, 50, 60, 70, 80, 90, 100, 150, 200, 250, 300, 350, 400, 450, 500, 550, 600, 650, 700, 750, 800 or 850; *y* is selected from 0.50, 0.60, 0.70, 0.75, 0.80, 0.85, 0.90, 0.91, 0.92, 0.93, 0.94, 0.95, 0.96, 0.97, 0.98, or 0.99; and, if *x·y* is not an integer, it is rounded up to the nearest integer.

These polypeptides include homologs, orthologs, allelic variants and mutants of SEQ ID NOs 1, 2, 4, 5 and 6. For instance, it is known to mutate natural Env sequences to improve resistance to proteases. The polypeptides also include fusion polypeptides, in which the Env sequence is fused to non-Env sequence. For instance, it is known to fuse Env sequences without the native leader peptide to leader peptides from non-Env proteins e.g. from tissue plasminogen activator.

Within category (ii), the degree of sequence identity may be greater than 50% (e.g. 60%, 70%, 75%, 80%, 85%, 90%, 91%, 92%, 93%, 94%, 95%, 96%, 97%, 98%, 99% or more). Identity between polypeptides is preferably determined by the Smith-Waterman homology search algorithm as implemented in the MPSRCH program (Oxford Molecular), using an affine gap search with parameters *gap open penalty=12* and *gap extension penalty= 1.*

Within category (iii), each substitution involves a single amino acid, each deletion preferably involves a single amino acid, and each insertion preferably involves a single amino acid. These changes may arise deliberately *(e.g.* by site-directed mutagenesis) or naturally *(e.g.* through virus evolution or through spontaneous mutation). The polypeptides in category (iii) may have one or more *(e.g.* 1, 2, 3, 4, 5, 6, 7, 8, 9, 10, *etc.)* single amino acid substitutions relative to SEQ ID NO: 1, 2, 4, 5 or 6. These polypeptides may have one or more *(e.g.* 1, 2, 3, 4, 5, 6, 7, 8, 9, 10, *etc.)* single amino acid deletions relative to SEQ ID NO: 1, 2, 4, 5 or 6. These polypeptide s may have one or more (e.g. 1, 2, 3, 4, 5, 6, 7, 8, 9, 10, *etc.)* single amino acid insertion relative to SEQ ID NO: 1, 2, 4, 5 or 6. The substitutions, insertions and/or deletions may be at separate locations or may be contiguous. Substitutions may be conservative *i.e*. replacements of one amino acid with another which has a related side chain. Genetically-encoded amino acids are generally divided into four families: (1) acidic *i.e.* aspartate, glutamate; (2) basic *i.e.* lysine, arginine, histidine; (3) non-polar *i.e.* alanine, valine, leucine, isoleucine, proline, phenylalanine, methionine, tryptophan; and (4) uncharged polar *i.e*. glycine, asparagine, glutamine, cysteine, serine, threonine, tyrosine. Phenylalanine, tryptophan, and tyrosine are sometimes classified jointly as aromatic amino acids. In general, substitution of single amino acids within these families does not have a major effect on the biological activity. Various substitutions have been described for use with Env polypeptides e.g. it is known to inactivate the cleavage site between gp120 and gp41 (e.g. by a Lys→Ser substitution) in order to provide a polypeptide that remains in full-length form, or to remove the 'clipping' site in the V3 loop [15], or to delete or substitute glycosylation sites, particularly N-glycosylation sites (*i.e.* asparagine residues).

Within category (iv), the value of n may be greater than 7 *e.g.* 8, 10, 12, 14, 16, 18, 20, 22, 24, 26, 28, 30, 35, 40, 45, 50, 60, 70, 80, 90, 100, 150, 200, 250, 300, 350, 400, 450, 500, 550, 600, 650, 700, 750, 800, 850 or more. The fragment may comprise at least one T-cell and/or B-cell epitope of the sequence. T- and B-cell epitopes can be identified empirically (e.g. using PEPSCAN [16,17] or similar methods), or they can be predicted (e.g. using the Jameson-Wolf antigenic index [18], matrix-based approaches [19], TEPITOPE [20], neural networks [21], OptiMer & EpiMer [22,23], ADEPT [24], Tsites [25], hydrophilicity [26], antigenic index [27] or the methods disclosed in ref. 28, *etc.).*

Within category (v), the preferred pairwise alignment algorithm is the Needleman-Wunsch global alignment algorithm [29], using default parameters (*e.g.* with Gap opening penalty = 10.0, and with Gap extension penalty = 0.5, using the EBLOSUM62 scoring matrix). This algorithm is conveniently implemented in the *needle* tool in the EMBOSS package [30].

Within this group of Env polypeptides that may be used with the invention, a preferred feature is that the polypeptide should retain the ability of natural Env to bind to CD4.

Env polypeptide is found in oligomeric form on the HIV virion, and preferred Env polypeptides used with the invention can also form oligomers, and in particular trimers. For instance, ΔV2 mutants of gp140 have been shown to form trimers [13] (sometimes referred to as 'o-gp140').

The Env protein may be used in combination with one or more further HIV antigens, such as Gag, Pol, Env, Tat, Rev, Nef, Vpr, Vpu and/or Vif. For instance, reference 1 uses combinations of Env *(e.g.* o-gp140) and Gag (e.g. p24). Similarly, reference 31 discloses the use of a combination of Env and Tat polypeptides, and reference 32 uses a complex of Env and Tat proteins for immunization. These and other mixtures can be used with the invention.

### The mucosal immunization(s)

The invention involves an initial mucosal immunization with HIV antigen(s). A patient may receive a single mucosal dose, or may receive a series of multiple mucosal doses (e.g. 2, 3, 4, 5, 6, 7, 8 or more mucosal doses). A preferred regimen involves 3 mucosal immunizations.

Where multiple doses are administered, each dose after the first dose will typically be administered at least 2 weeks after the previous one *e.g.* ≥3 weeks apart, ≥4 weeks apart, ≥6 weeks apart, ≥8 weeks apart, ≥12 weeks apart, *etc.* A typical 3-dose schedule may be at 0, 4 and 8 weeks, where 0 is the time of the first dose.

Routes for mucosal administration of compositions include intranasal [33-35], intragastric, rectal, vaginal, peroral [36], pulmonary, intestinal, intradermal, transdermal, buccal, sublingual, *etc.* Where more than one mucosal immunization is given, each of them is preferably via the same route. The preferred route for mucosal administration is the intranasal route, as it offers easy access with relatively simple devices that have already been mass produced. The composition of the invention may thus be presented for intranasal administration, such as by nasal spray, nasal drops, gel or powder (e.g. refs 37 & 38). Mucosal administration may also involve presenting the composition in the form of tablets or capsules (optionally enteric-coated), liquid, transgenic plant material, drops, inhalers, aerosol, suppositories, pessaries, *etc.* (see also ref. 39, and Chapter 17 of ref. 57).

Antigens given by a mucosal route will typically be administered in combination with (e.g. in admixture with) a suitable adjuvant. Adjuvants that are active via mucosal routes include, but are not limited to:
- Bacterial ADP-ribosylating toxins *(e.g.* the *E.coli* heat labile enterotoxin "LT", cholera toxin "CT", or pertussis toxin "PT") and detoxified derivatives thereof, such as the mutant toxins known as LT-K63 and LT-R72 [40]. Mutagenesis can be used to remove the toxic enzymatic activity of these toxins without removing their adjuvant activity. The use of detoxified ADP-ribosylating toxins as mucosal adjuvants is described in reference 41.
- Microparticles (*i.e.* a particle of ∼100nm to ∼150µm in diameter, more preferably ∼200nm to ∼30µm in diameter, and most preferably ∼500nm to ∼10µm in diameter) formed from materials that are biodegradable and non-toxic (e.g. a poly(α-hydroxy acid), a polyhydroxybutyric acid, a polyorthoester, a polyanhydride, a polycaprolactone *etc.,* such as poly(lactide-co-glycolide) *etc.)* optionally treated to have a negatively-charged surface (e.g. with SDS) or a positively-charged surface (e.g. with a cationic detergent, such as CTAB).

- A polyoxyethylene ether or a polyoxyethylene ester [42]. Such adjuvant formulations further include polyoxyethylene sorbitan ester surfactants in combination with an octoxynol [43] as well as polyoxyethylene alkyl ethers or ester surfactants in combination with at least one additional non-ionic surfactant such as an octoxynol [44]. Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.
- Bioadhesives [45] and mucoadhesives, such as esterified hyaluronic acid microspheres [46], chitosan [47] and its derivatives [48], cross-linked derivatives of poly(acrylic acid), polyvinyl alcohol, polyvinyl pyrollidone, polysaccharides and carboxymethylcellulose.
- An immunostimulatory oligonucleotide (e.g. a CpG oligonucleotide) and a saponin [49].
- Liposomes [chapters 13 & 14 of ref. 57]. Examples of liposome formulations suitable for use as adjuvants are described in refs. 50-52.
- Aminoalkyl glucosaminide phosphate derivatives, such as RC-529 [53,54].
- A phosphazene, such as poly[di(carboxylatophenoxy)phosphazene] ("PCPP") as described, for example, in references 55 and 56.

Compositions may include two or more of said adjuvants.

Other adjuvants that are active via mucosal routes are also available (e.g. see chapter 7 of ref. 57).

A preferred class of mucosal adjuvants is the group of ADP-ribosylating toxins. Within this group, detoxified toxins are preferred. Detoxified mutants of LT and CT are preferred. In one embodiment, LT having a mutation at residue Ala-72 (e.g. an Arg substitution [58], to give 'LT-R72') can be used. In another embodiment, LT having a mutation at residue Ser-63 (e.g. a Lys substitution [Chapter 5 of ref. 59] to give 'LT-K63', or a Tyr substitution [60]). Numbering of these residues is according to reference 61. The LT-K63 mutant is particularly suitable for use with the invention.

Thus a preferred mucosal immunization used with the invention involves multiple doses (e.g. 3) by the intranasal route using LT-K63 adjuvant. A typical intranasal composition will include between 10µg and 50µg of LT adjuvant per dose e.g. about 30µg of LT-K63 per dose.

### The parenteral immunization(s)

After an initial mucosal immunization, envelope antigen is administered parenterally. A patient may receive a single parenteral dose, or may receive a series of multiple parenteral doses (e.g. 2, 3, 4, 5, 6, 7, 8 or more mucosal doses). A preferred regimen involves 2 parenteral immunizations.

The first (or only) parenteral dose will typically be administered at least 2 weeks after the final dose in the mucosal series e.g. ≥3 weeks after, ≥4 weeks after, ≥6 weeks after, ≥8 weeks after, ≥12 weeks after, etc. The first parenteral dose may be administered, for instance, about 8 weeks after the final (e.g. third) mucosal dose.

Where multiple parenteral doses are administered, each dose after the first parenteral dose will typically be administered at least 2 weeks after the previous one e.g. ≥3 weeks apart, ≥4 weeks apart, ≥6 weeks apart, ≥8 weeks apart, ≥12 weeks apart, *etc.* A typical 2-dose schedule may be at 0 and 12 weeks, where 0 is the time of the first parenteral dose (e.g. at 16 & 28 weeks relative to the first mucosal dose.)

Routes for parenteral administration of compositions include subcutaneous, intravenous, intramuscular, etc. Administration via these routes will typically be by injection. Where more than one parenteral immunization is given, each of them is preferably via the same route. The preferred route for parenteral administration is by intramuscular injection.

Antigens given by a parenteral route will typically be administered in combination with (e.g. in admixture with) a suitable adjuvant. Particularly suitable adjuvants are capable of inducing (eliciting) a Th1-type immune response in a subject. Such adjuvants are also referred to herein as Th1 adjuvants. The distinction between Th1 and Th2 T helper cells is well known. Th1 and Th2 adjuvants cause an immune response to a co-administered antigen to be biased towards, respectively, a Th1-type or a Th2-type response. Thus Th1 adjuvants result in the production of antigen-specific T cells that release cytokines such as IL-2 and interferon-γ (leading to IgG2a antibodies), whereas Th2 adjuvants result in the production of antigen-specific T cells that release cytokines such as IL-4 (leading to IgG1).

Th1-type responses are naturally linked to bacterial infections, and so adjuvants used with the invention generally include substances that mimic a bacterial substance. Th1 adjuvants may be modulators and/or agonists of Toll-Like Receptors (TLR). For example, they may be agonists of one or more of the human TLR1, TLR2, TLR3, TLR4, TLR7, TLR8, and/or TLR9 proteins. Preferred agents are agonists of TLR7 (e.g. imidazoquinolines) and/or TLR9 (e.g. CpG oligonucleotides). These agents are useful for activating innate immunity pathways.

Adjuvants that are active for parenterally administered antigens include, but are not limited to:
- An oil-in-water emulsion, as described in more detail below.
- An immunostimulatory oligonucleotide, such as one containing a CpG motif (a dinucleotide sequence containing an unmethylated cytosine linked by a phosphate bond to a guanosine), a TpG motif [62],a double-stranded RNA, an oligonucleotide containing a palindromic sequence, or an oligonucleotide containing a poly(dG) sequence. Immunostimulatory oligonucleotides can include nucleotide modifications/analogs such as phosphorothioate modifications and can be double-stranded or (except for RNA) single-stranded. References 63 to 65 disclose possible analog substitutions *e.g.* replacement of guanosine with 2'-deoxy-7-deazaguanosine. The adjuvant effect of CpG oligonucleotides is further discussed in refs. 66-71. A CpG sequence may be directed to TLR9, such as the motif GTCGTT or TTCGTT [72]. The CpG sequence may be specific for inducing a Th1 immune response, such as a CpG-A ODN (oligodeoxynucleotide), or it may be more specific for inducing a B cell response, such a CpG-B ODN. CpG-A and CpG-B ODNs are discussed in refs. 73-75. Preferably, the CpG is a CpG-A ODN. Preferably, the CpG oligonucleotide is constructed so that the 5' end is accessible for receptor recognition. Optionally, two CpG oligonucleotide sequences may be attached at their 3' ends to form "immunomers". See, for example, references 72 & 76-78. A useful CpG adjuvant is CpG7909, also known as ProMune™ (Coley Pharmaceutical Group, Inc.). Immunostimulatory oligonucleotides will typically comprise at least 20 nucleotides. They may comprise fewer than 100 nucleotides.
- 3-O-deacylated monophosphoryl lipid A ('3dMPL', also known as 'MPL™') [79-82]. 3dMPL has been prepared from a heptoseless mutant of *Salmonella minnesota,* and is chemically similar to lipid A but lacks an acid-labile phosphoryl group and a base-labile acyl group. Preparation of 3dMPL was originally described in reference 83. 3dMPL can take the form of a mixture of related molecules, varying by their acylation *(e.g.* having 3, 4, 5 or 6 acyl chains, which may be of different lengths). The two glucosamine (also known as 2-deoxy-2-amino-glucose) monosaccharides are N-acylated at their 2-position carbons (*i.e.* at positions 2 and 2'), and there is also O-acylation at the 3' position. 3dMPL promotes a Th1-type response [84].
- An imidazoquinoline compound, such as Imiquimod ("R-837") [85,86], Resiquimod ("R-848") [87], and their analogs; and salts thereof (e.g. the hydrochloride salts). Further details about immunostimulatory imidazoquinolines can be found in references 88 to 92. These compounds shift responses towards Th1.
- A thiosemicarbazone compound, such as those disclosed in reference 93. Methods of formulating, manufacturing, and screening for active compounds are also described in reference 93. The thiosemicarbazones are particularly effective in the stimulation of human peripheral blood mononuclear cells for the production of cytokines, such as TNF-α.
- A tryptanthrin compound, such as those disclosed in reference 94. Methods of formulating, manufacturing, and screening for active compounds are also described in reference 94. The thiosemicarbazones are particularly effective in the stimulation of human peripheral blood mononuclear cells for the production of cytokines, such as TNF-α.
- A mineral-containing composition, including calcium salts and aluminum salts (or mixtures thereof). Calcium salts include calcium phosphate (*e.g.* the "CAP" particles disclosed in ref. 95). Aluminum salts include hydroxides, phosphates, sulfates, etc., with the salts taking any suitable form (e.g. gel, crystalline, amorphous, etc.). Adsorption to these salts is preferred. The mineral containing compositions may also be formulated as a particle of metal salt [96]. Aluminum salt adjuvants are described in more detail below.
- A nucleoside analog, such as: (a) Isatorabine (ANA-245; 7-thia-8-oxoguanosine): 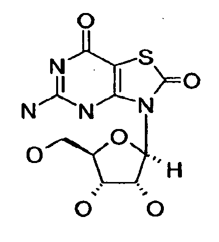 and prodrugs thereof; (b) ANA975; (c) ANA-025-1; (d) ANA380; (e) the compounds disclosed in references 97 to 99; (f) a compound having the formula: 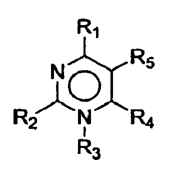 wherein:
   R₁ and R₂ are each independently H, halo, -NRₐR_{b}, -OH, C₁₋₆ alkoxy, substituted C₁₋₆ alkoxy, heterocyclyl, substituted heterocyclyl, C₆₋₁₀ aryl, substituted C₆₋₁₀ aryl, C₁₋₆ alkyl, or substituted C₁₋₆ alkyl;
   R₃ is absent, H, C₁₋₆ alkyl, substituted C₁₋₆ alkyl, C₆₋₁₀ aryl, substituted C₆₋₁₀ aryl, heterocyclyl, or substituted heterocyclyl;
   R₄ and R₅ are each independently H, halo, heterocyclyl, substituted heterocyclyl, -C(O)-R_{d}, C₁₆ alkyl, substituted C₁₋₆ alkyl, or bound together to form a 5 membered ring as in R₄₋₅: 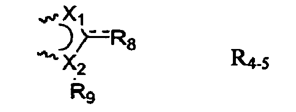 the binding being achieved at the bonds indicated by a
   X₁ and X₂ are each independently N, C, O, or S;
   R₈ is H, halo, -OH, C₁₋₆ alkyl, C₂₋₆ alkenyl, C₂₋₆ alkynyl, -OH, -NRₐR_{b}, -(CH₂)ₙ-O-R_{c}, -O-(C₁₋₆ alkyl), -S(O)ₚRₑ, or -C(O)-R_{d};
   R₉ is H, C₁₋₆ alkyl, substituted C₁₋₆ alkyl, heterocyclyl, substituted heterocyclyl or R₉ₐ, wherein R₉ₐ is: 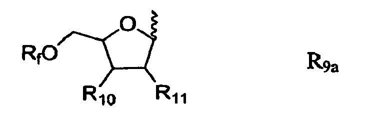 the binding being achieved at the bond indicated by a
   R₁₀ and R₁₁ are each independently H, halo, C₁₋₆ alkoxy, substituted C₁₋₆ alkoxy, - NRₐR_{b}, or -OH;
   each Rₐ and R_{b} is independently H, C₁₋₆ alkyl, substituted C₁₋₆ alkyl, -C(O)R_{d}, C₆₋₁₀ aryl;
   each R_{c} is independently H, phosphate, diphosphate, triphosphate, C₁₋₆ alkyl, or substituted C₁₋₆ alkyl;
   each R_{d} is independently H, halo, C₁₋₆ alkyl, substituted C₁₋₆ alkyl, C₁₋₆ alkoxy, substituted C₁₋₆ alkoxy, -NH₂, -NH(C₁₋₆ alkyl), -NH(substituted C₁₋₆ alkyl), -N(C₁₋₆ alkyl)₂, -N(substituted C₁₋₆ alkyl)₂, C₆₋₁₀ aryl, or heterocyclyl;
   each Rₑ is independently H, C₁₋₆ alkyl, substituted C₁₋₆ alkyl, C₆₋₁₀ aryl, substituted C₆₋₁₀ aryl, heterocyclyl, or substituted heterocyclyl;
   each R_{f} is independently H, C₁₋₆ alkyl, substituted C₁₋₆ alkyl, -C(O)R_{d}, phosphate, diphosphate, or triphosphate;
   each n is independently 0, 1, 2, or 3;
   each p is independently 0, 1, or 2; or
   or (g) a pharmaceutically acceptable salt of any of (a) to (f), a tautomer of any of (a) to (f), or a pharmaceutically acceptable salt of the tautomer.
- Loxoribine (7-allyl-8-oxoguanosine) [100].
- Compounds disclosed in reference 101, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds [102,103], Hydrapthalamide compounds, Benzophenone compounds, Isoxazole compounds, Sterol compounds, Quinazilinone compounds, Pyrrole compounds [104], Anthraquinone compounds, Quinoxaline compounds, Triazine compounds, Pyrazalopyrimidine compounds, and Benzazole compounds [105].
- Compounds disclosed in reference 106, including 3,4-di(1H-indol-3-yl)-1H-pyrrole-2,5-diones, staurosporine analogs, derivatized pyridazines, chromen-4-ones, indolinones, quinazolines, and nucleoside analogs.
- An aminoalkyl glucosaminide phosphate derivative, such as RC-529 (see above). These derivatives stimulate Th1 responses [107].
- A phosphazene, as described above.
- Small molecule immunopotentiators (SMIPs) such as:
   N2-methyl-1-(2-methylpropyl)-1H-imidazo[4,5-c]quinoline-2,4-diamine
   N2,N2-dimethyl-1-(2-methylpropyl)-1H-imidazo[4,5-c]quinoline-2,4-diamine
   N2-ethyl-N2-methyl-1-(2-methylpropyl)-1H-imidazo[4,5-c]quinoline-2,4-diamine
   N2-methyl-1-(2-methylpropyl)-N2-propyl-1H-imidazo[4,5-c] quinoline-2,4-diamine
   1-(2-methylpropyl)-N2-propyl-1H-imidazo[4,5-c]quinoline-2,4-diamine
   N2-butyl-1-(2-methylpropyl)-1H-imidazo[4,5-c]quinoline-2,4-diamine
   N2-butyl-N2-methyl-1-(2-methylpropyl)-1-H-imidazo[4,5-c]quinoline-2,4-diamine
   N2-methyl-1-(2-methylpropyl)-N2-pentyl-1H-imidazo[4,5-c]quinoline-2,4-diamine
   N2-methyl-1-(2-methylpropyl)-N2-prop-2-enyl-1H-imidazo[4,5-c]quinoline-2,4-diamine
   1-(2-methylpropyl)-2-[(phenylmethyl)thio]-1H-imidazo[4,5-c]quinolin-4-amine
   1-(2-methylpropyl)-2-(propylthio)-1H-imidazo[4,5-c]quinolin-4-amine
   2-[[4-amino-1-(2-methylpropyl)-1H-imidazo[4,5-c]quinolin-2-yl](methyl)aminolethanol
   2-[[4-amino-1-(2-methylpropyl)-1H-imidazo[4,5-c]quinolin-2-yl](methyl)amino]ethyl acetate
   4-amino-1-(2-methylpropyl)-1,3-dihydro-2H-imidazo[4,5-c]quinolin-2-one
   N2-butyl-1-(2-methylpropyl)-N4,N4-bis(phenylmethyl)-11H-imidazo[4,5-c]quinoline-2,4-diamine
   N2-butyl-N2-methyl-1-(2-methylpropyl)-N4,N4-bis(phenylmethyl)-1H-imidazo[4,5-c]quinoline-2,4-diamine
   N2-methyl-1-(2-methylpropyl)-N4,N4-bis(phenylmethyl)-1H-imidazo[4,5-c]quinoline-2,4-diamine
   N2,N2-dimethyl-1-(2-methylpropyl)-N4,N4-bis(phenylmethyl)-1H-imidazo[4,5-c]quinoline-2,4-diamine
   1-{4-amino-2-[methyl(propyl)amino]-1H-imidazo[4,5-c]quinolin-1-yl}-2-methylpropan-2-ol
   1-[4-amino-2-(propylamino)-1H-imidazo[4,5-c]quinolin-1-yl]-2-methylpropan-2-ol
   N4,N4-dibenzyl-1-(2-methoxy-2-methylpropyl)-N2-propyl-1H-imidazo[4,5-c]quinoline-2,4-diamine.
- Saponins [chapter 22 of ref. 136], which are a heterologous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs. QS21 is marketed as Stimulon™. Saponin compositions have been purified using HPLC and RP-HPLC. Specific purified fractions using these techniques have been identified, including QS7, QS 17, QS18, QS21, QH-A, QH-B and QH-C. Preferably, the saponin is QS21. A method of production of QS21 is disclosed in ref. 108. Saponin formulations may also comprise a sterol, such as cholesterol [109]. Combinations of saponins and cholesterols can be used to form unique particles called immunostimulating complexs (ISCOMs) [chapter 23 of ref. 136]. ISCOMs typically also include a phospholipid such as phosphatidylethanolamine or phosphatidylcholine. Any known saponin can be used in ISCOMs. Preferably, the ISCOM includes one or more of QuilA, QHA & QHC. ISCOMs are further described in refs. 109-111. Optionally, the ISCOMS may be devoid of additional detergent [112]. A review of the development of saponin based adjuvants can be found in refs. 113 & 114. ISCOMs and free QS21 have both been reported to upregulate Th1 responses.
- Bacterial ADP-ribosylating toxins (see above). The use of detoxified ADP-ribosylating toxins as parenteral adjuvants is described in reference 115. Some mutants have been reported to induce a polarized Th2-type response (e.g. LT-R72 in ref. 116; but *cf.* ref. 117), but others have been reported to induce mixed Th1/Th2-type responses (e.g. LT-K63) or Th1-type responses (e.g. LT-G192). The Th1/Th2 balance achieved by any particular mutant when given with an antigen by a chosen route and schedule can easily be assessed.
- Microparticles (*i.e.* a particle of ∼100nm to ∼150µm in diameter, more preferably ∼200nm to ∼30um in diameter, and most preferably ∼500nm to ∼10µm in diameter) formed from materials that are biodegradable and non-toxic (e.g. a poly(α-hydroxy acid), a polyhydroxybutyric acid, a polyorthoester, a polyanhydride, a polycaprolactone, *etc.),* with poly(lactide-co-glycolide) being preferred, optionally treated to have a negatively-charged surface (e.g. with SDS) or a positively-charged surface (e.g. with a cationic detergent, such as CTAB). Encapsulation of antigens into PLG microparticles has been reported to favor a Th1-type response when compared to MF59 [118].
- Liposomes (Chapters 13 & 14 of ref. 136). Liposomes can elicit strong Th1 responses, particularly cationic liposomes containing mycobacterial lipids [119].
- A calcium salt, such as calcium phosphate (e.g. the "CAP" particles disclosed in ref. 120). Calcium salts have been reported to provide Th1 responses [121].
- Muramyl peptides, such as N-acetylmuramyl-L-threonyl-D-isoglutamine ("thr-MDP"), N-acetyl-normuramyl-L-alanyl-D-isoglutamine (nor-MDP), N-acetylglucsaminyl-N-acetylmuramyl-L-Al-D-isoglu-L-Ala-dipalmitoxy propylamide ("DTP-DPP", or "Theramide™), N-acetylmuramyl-L-alanyl-D-isoglutaminyl-L-alanine-2-(1'-2'dipalmitoyl-sn-glycero-3-hydroxyphosphoryloxy)-ethylamine ("MTP-PE").
- An outer membrane protein proteosome preparation prepared from a first Gram-negative bacterium in combination with a liposaccharide (LPS) preparation derived from a second Gram-negative bacterium, wherein the outer membrane protein proteosome and LPS preparations form a stable non-covalent adjuvant complex. Such complexes include "IVX-908", a complex comprised of *Neisseria meningitidis* outer membrane and LPS.
- Methyl inosine 5'-monophosphate ("MIMP") [122].
- A polyhydroxlated pyrrolizidine compound [123], such as one having formula: 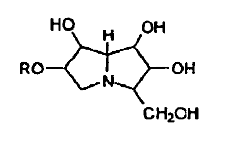 where R is selected from the group comprising hydrogen, straight or branched, unsubstituted or substituted, saturated or unsaturated acyl, alkyl (e.g. cycloalkyl), alkenyl, alkynyl and aryl groups, or a pharmaceutically acceptable salt or derivative thereof. Examples include, but are not limited to: casuarine, casuarine-6-α-D-glucopyranose, 3-*epi*-casuarine, 7-epi-casuarine, 3,7-diepi-casuarine, etc. These compounds enhance Th1 responses.
- A gamma inulin [124] or derivative thereof, such as algammulin. These adjuvants can promote both Th1 and Th2 immune responses [125].

A vitamin E compound. Vitamin E has a significant impact on the expression of genes involved in the Th1/Th2 balance, and vitamin E stimulation of immune cells can directly lead to increased IL-2 production (*i.e.* a Th1-type response) [126]. Natural vitamin E exists in eight different forms or isomers: four tocopherols and four tocotrienols. All isomers have a chromanol ring, with a hydroxyl group which can donate a hydrogen atom to reduce free radicals and a hydrophobic side chain which allows for penetration into biological membranes. There is an a, β, γ and δ form of both the tocopherols and tocotrienols, determined by the number of methyl groups on the chromanol ring. Each form has its own biological activity, the measure of potency or functional use in the body. The tocopherols can take several forms e.g. different salts and/or isomers. Salts include organic salts, such as succinate, acetate, nicotinate, etc. D-α-tocopherol and DL-α-tocopherol can both be used. A preferred α-tocopherol is DL-α-tocopherol, and the preferred salt of this tocopherol is the succinate. As vitamin E compounds are usually oils, they can conveniently be included as a component in an oil-in-water emulsion, and oil-in-water emulsions containing tocopherols are reported in reference **Error! Bookmark not defined.** to be Th1-inducing adjuvants.
- A compound of formula I, II or III, or a salt thereof: 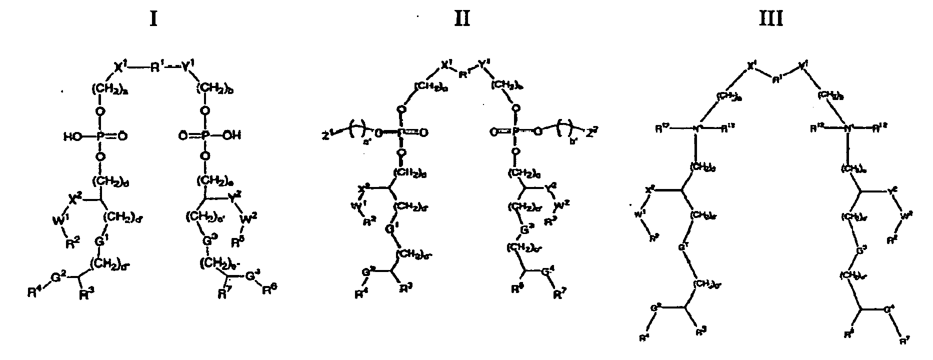 as defined in reference 127, such as 'ER 803058', 'ER 803732', 'ER 804053', ER 804058', 'ER 804059', 'ER 804442', 'ER 804680', 'ER 804764', ER 803022 or 'ER 804057' e.g.: 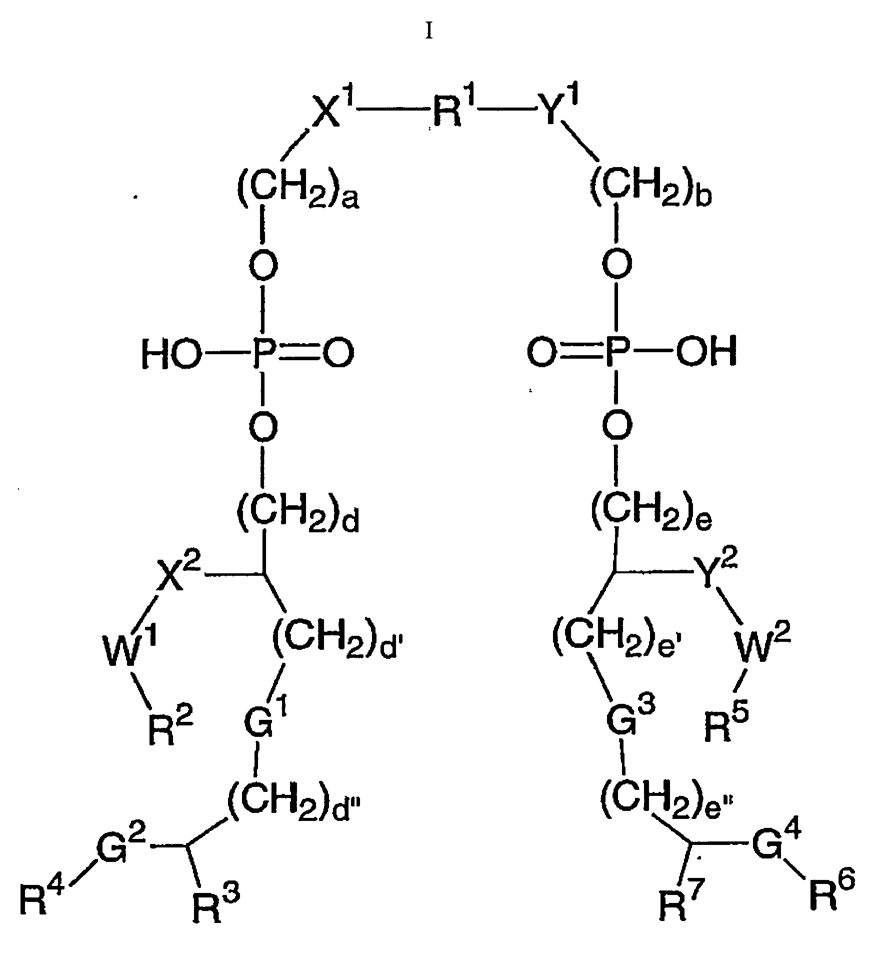 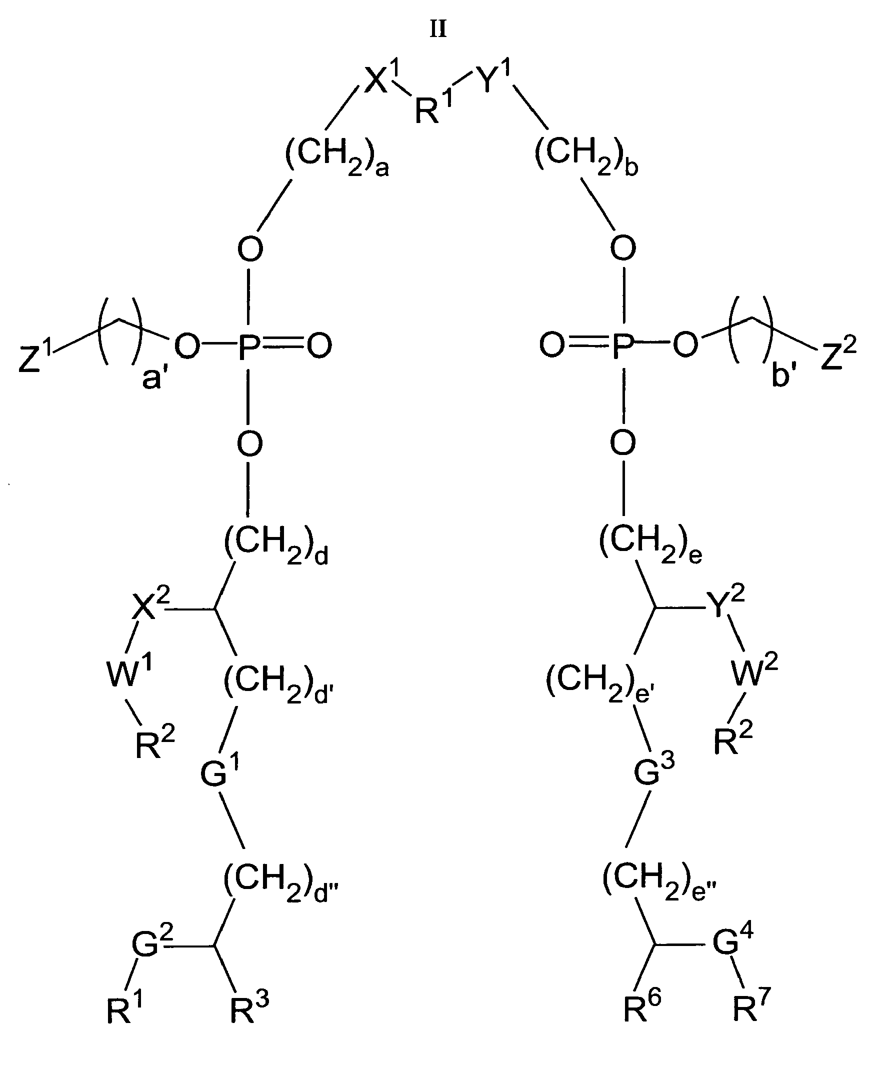 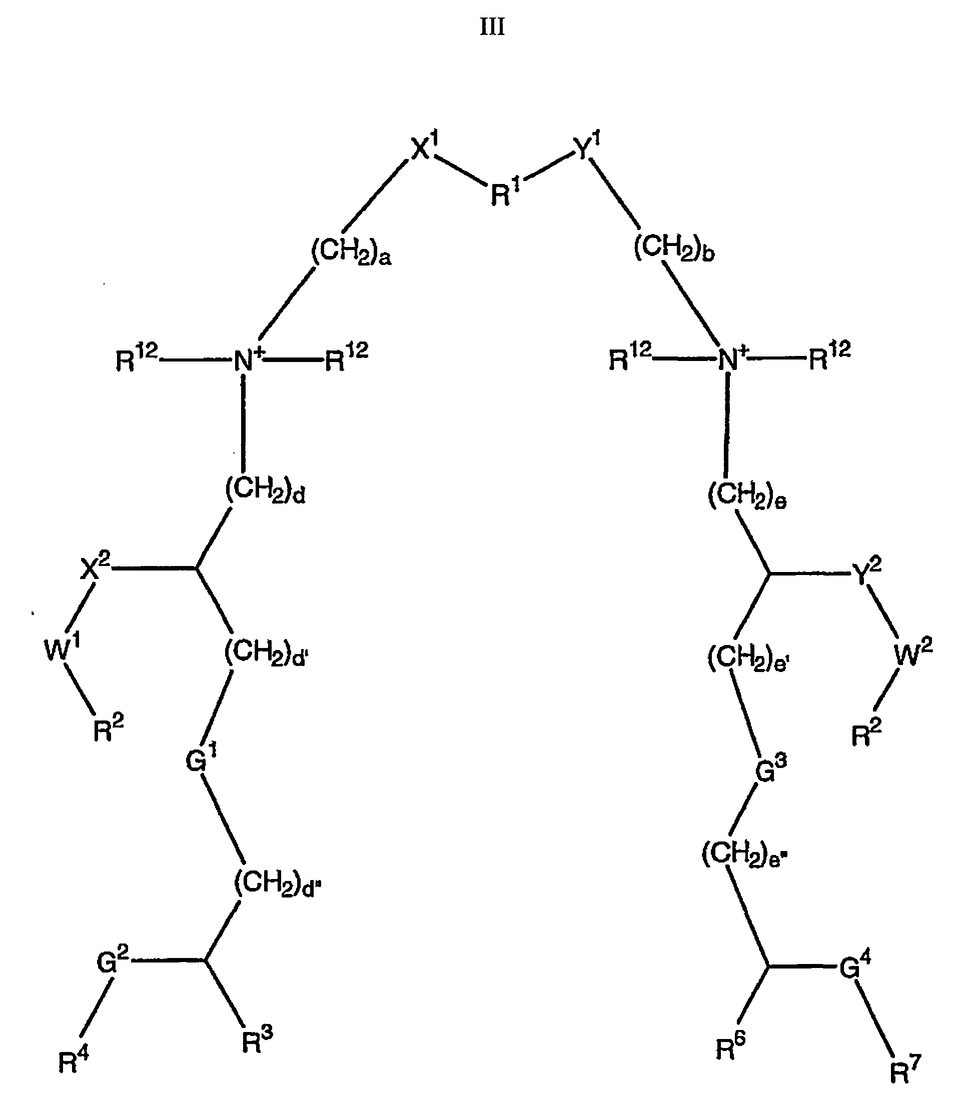 as defined in reference 127, such as 'ER 803058', 'ER 803732', 'ER 804053', ER 804058', 'ER 804059', 'ER 804442', 'ER 804680', 'ER 804764', ER 803022 or 'ER 804057' e.g.:
   where R is selected from the group comprising hydrogen, straight or branched, unsubstituted or substituted, saturated or unsaturated acyl, alkyl (e.g. cycloalkyl), alkenyl, alkynyl and aryl groups, or a pharmaceutically acceptable salt or derivative thereof. Examples include, but are not limited to: casuarine, casuarine-6-α-D-glucopyranose, 3-*epi*-casuarine, 7-epi-casuarine, 3,7-diepi-casuarine, etc. These compounds enhance Th1 responses.
- A gamma inulin [124] or derivative thereof, such as algammulin. These adjuvants can promote both Th1 and Th2 immune responses [125].

A vitamin E compound. Vitamin E has a significant impact on the expression of genes involved in the Th1/Th2 balance, and vitamin E stimulation of immune cells can directly lead to increased IL-2 production (*i.e.* a Th1-type response) [126]. Natural vitamin E exists in eight different forms or isomers: four tocopherols and four tocotrienols. All isomers have a chromanol ring, with a hydroxyl group which can donate a hydrogen atom to reduce free radicals and a hydrophobic side chain which allows for penetration into biological membranes. There is an a, β, γ and δ form of both the tocopherols and tocotrienols, determined by the number of methyl groups on the chromanol ring. Each form has its own biological activity, the measure of potency or functional use in the body. The tocopherols can take several forms e.g. different salts and/or isomers. Salts include organic salts, such as succinate, acetate, nicotinate, etc. D-α-tocopherol and DL-α-tocopherol can both be used. A preferred α-tocopherol is DL-α-tocopherol, and the preferred salt of this tocopherol is the succinate. As vitamin E compounds are usually oils, they can conveniently be included as a component in an oil-in-water emulsion, and oil-in-water emulsions containing tocopherols are reported in reference **Error! Bookmark not defined.** to be Th1-inducing adjuvants.
- A compound of formula I, II or III, or a salt thereof: 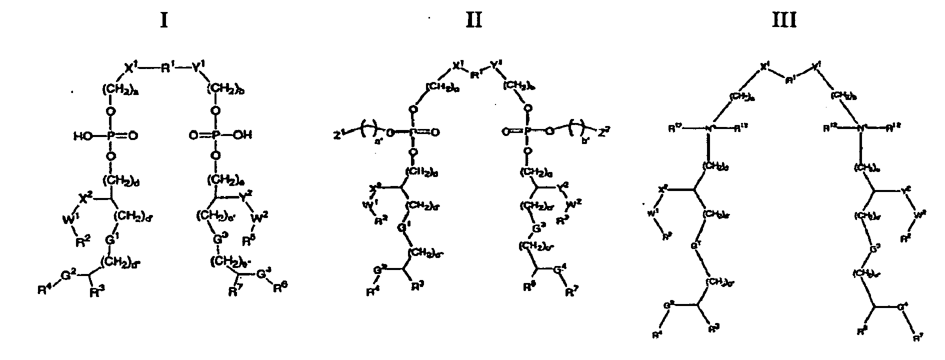 as defined in reference 127, such as 'ER 803058', 'ER 803732', 'ER 804053', ER 804058', 'ER 804059', 'ER 804442', 'ER 804680', 'ER 804764', ER 803022 or 'ER 804057' e.g.: 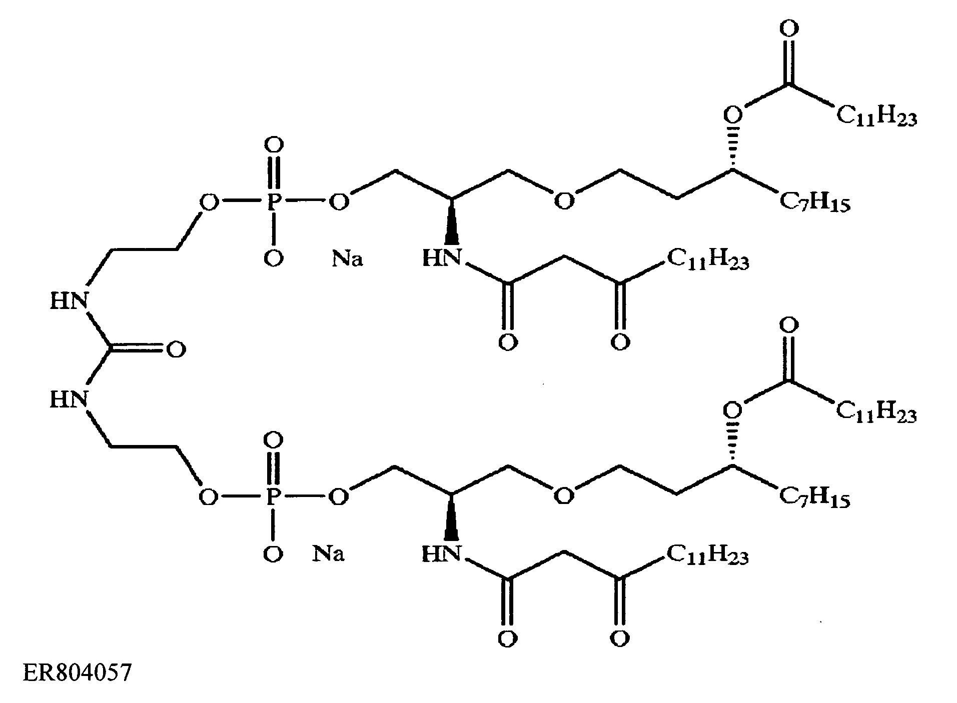 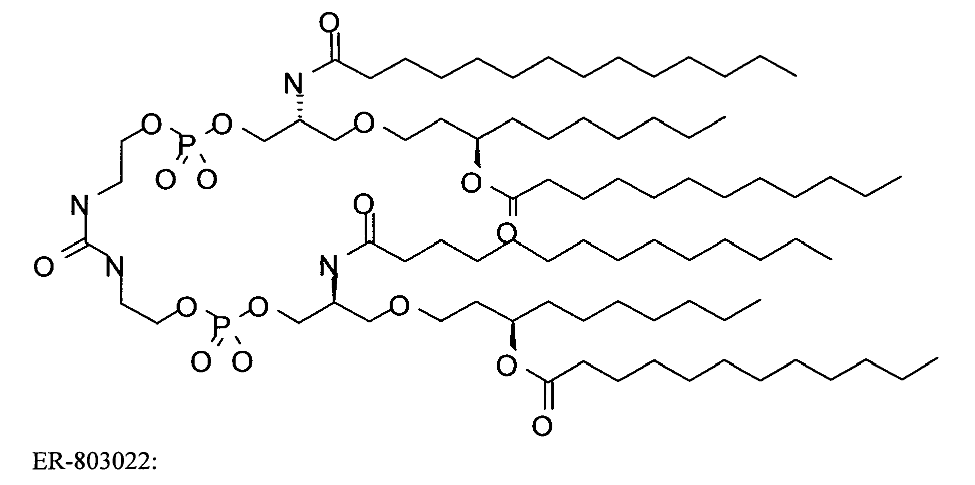
- Derivatives of lipid A from Escherichia coli such as OM-174 (described in refs. 128 & 129).
- A formulation of a cationic lipid and a (usually neutral) co-lipid, such as aminopropyl-dimethyl-myristoleyloxy-propanaminium bromide-diphytanoylphosphatidyl-ethanolamine ("Vaxfectin™") or aminopropyl-dimethyl-bis-dodecyloxy-propanaminium bromide-dioleoylphosphatidyl-ethanolamine ("GAP-DLRIE:DOPE"). Formulations containing (±)-N-(3-aminopropyl)-N,N-dimethyl-2,3-bis(syn-9-tetradeceneyloxy)-1-propanaminium salts are preferred [130].
- Compounds containing lipids linked to a phosphate-containing acyclic backbone, such as the TLR4 antagonist EE564 [131, 132]:

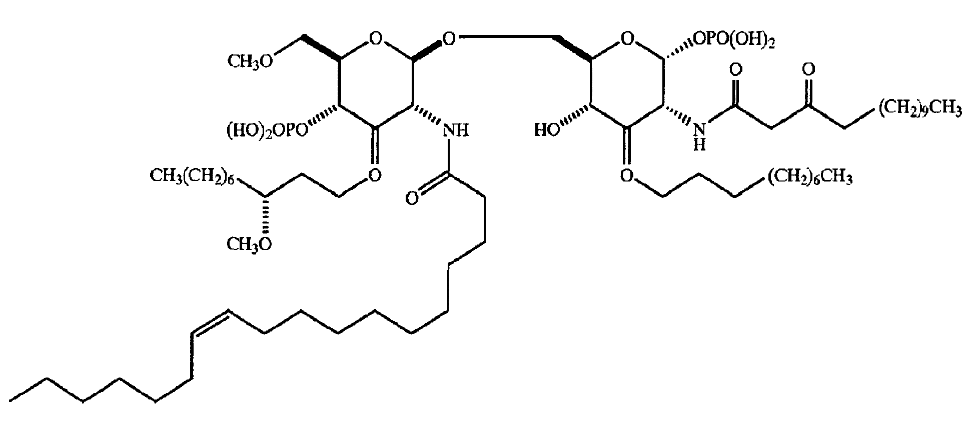

Compositions may include two or more of said adjuvants.

These and other adjuvant-active substances are discussed in more detail in references 136 & 137.

A preferred class of parenteral adjuvants is the group of oil-in-water emulsions, as described in more detail below. Within this group, squalene-containing emulsions are preferred, with the MF59 adjuvant being particularly suitable.

Thus a preferred parenteral immunization used with the invention involves multiple doses (e.g. 2) by intramuscular injection using a MF59 adjuvant.

Although the invention begins with mucosal administration, followed by parenteral administration, it does not exclude situations where mucosal administration is used again after parenteral administration. For example, a patient might receive a mucosal dose, a parenteral dose, and then another mucosal dose.

### Oil-in-water emulsion adjuvants

Oil-in-water emulsions are particularly useful as adjuvants. Various such emulsions are known, and they typically include at least one oil and at least one surfactant, with the oil(s) and surfactant(s) being biodegradable (metabolisable) and biocompatible. The oil droplets in the emulsion are generally less than 5µm in diameter, and may even have a sub-micron diameter, with these small sizes being achieved with a microfluidizer to provide stable emulsions. Droplets with a size less than 220nm are preferred as they can be subjected to filter sterilization.

The invention can be used with oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used e.g. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols (see below). Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X-100, or t-octylphenoxypolyethoxyethanol) being of particular interest; (octylphenoxy)polyethoxyethanol (IGEPAL CA-630/NP-40); phospholipids such as phosphatidylcholine (lecithin); polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Preferred surfactants for including in the emulsion are Tween 80 (polyoxyethylene sorbitan monooleate), Span 85 (sorbitan trioleate), lecithin and Triton X-100. Mixtures of surfactants can be used e.g. Tween 80/Span 85 mixtures.

Oil-in-water emulsion adjuvants useful with the invention include, but are not limited to:
● A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [133-135], as described in more detail in Chapter 10 of ref. 136 and chapter 12 of ref. 137. The MF59 emulsion advantageously includes citrate ions e.g. 10mM sodium citrate buffer.
● An emulsion of squalene, a tocopherol, and Tween 80. The emulsion may include phosphate buffered saline. It may also include Span 85 (e.g. at 1%) and/or lecithin. These emulsions may have from 2 to 10% squalene, from 2 to 10% tocopherol and from 0.3 to 3% Tween 80, and the weight ratio of squalene:tocopherol is preferably ≤1 as this provides a more stable emulsion. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets e.g. with an average diameter of between 100 and 250nm, preferably about 180nm.
● An emulsion of squalene, a tocopherol, and a Triton detergent (e.g. Triton X-100).
● An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [138] (0.05-1% Thr-MDP, 5% squalane, 2.5% Pluronic L121 and 0.2% polysorbate 80). It can also be used without the Thr-MDP, as in the "AF" adjuvant [139] (5% squalane, 1.25% Pluronic L121 and 0.2% polysorbate 80). Microfluidisation is preferred.
● An emulsion having from 0.5-50% of an oil, 0.1-10% of a phospholipid, and 0.05-5% of a non-ionic surfactant. As described in reference 140, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
● A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 141, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
● An emulsion in which a saponin (e.g. QuilA or QS21) and a sterol (e.g. a cholesterol) are associated as helical micelles [142].

Squalene-containing emulsions are preferred, with the MF59 adjuvant being particularly suitable. Thus a preferred parenteral immunization used with the invention involves multiple doses (e.g. 2) by intramuscular injection using a MF59 adjuvant.

The emulsions may be mixed with antigen extemporaneously, at the time of delivery. Thus the adjuvant and antigen may be kept separately in a packaged or distributed vaccine, ready for final formulation at the time of use. The antigen will generally be in an aqueous form, such that the vaccine is finally prepared by mixing two liquids. The volume ratio of the two liquids for mixing can vary (e.g. between 5:1 and 1:5) but is generally about 1:1.

### Aluminum salt adjuvants

The adjuvants known as aluminum hydroxide and aluminum phosphate may be used. These names are conventional, but are used for convenience only, as neither is a precise description of the actual chemical compound which is present (*e.g*. see chapter 9 of reference 136). The invention can use any of the "hydroxide" or "phosphate" adjuvants that are in general use as adjuvants.

The adjuvants known as "aluminum hydroxide" are typically aluminum oxyhydroxide salts, which are usually at least partially crystalline. Aluminum oxyhydroxide, which can be represented by the formula AlO(OH), can be distinguished from other aluminum compounds, such as aluminum hydroxide Al(OH)₃, by infrared (IR) spectroscopy, in particular by the presence of an adsorption band at 1070cm⁻¹ and a strong shoulder at 3090―3100cm⁻¹ [chapter 9 of ref. 136]. The degree of crystallinity of an aluminum hydroxide adjuvant is reflected by the width of the diffraction band at half height (WHH), with poorly-crystalline particles showing greater line broadening due to smaller crystallite sizes. The surface area increases as WHH increases, and adjuvants with higher WHH values have been seen to have greater capacity for antigen adsorption. A fibrous morphology (e.g. as seen in transmission electron micrographs) is typical for aluminum hydroxide adjuvants. The pI of aluminum hydroxide adjuvants is typically about 11 *i.e.* the adjuvant itself has a positive surface charge at physiological pH. Adsorptive capacities of between 1.8-2.6 mg protein per mg Al⁺⁺⁺ at pH 7.4 have been reported for aluminum hydroxide adjuvants.

The adjuvants known as "aluminum phosphate" are typically aluminum hydroxyphosphates, often also containing a small amount of sulfate (i.e. aluminum hydroxyphosphate sulfate). They may be obtained by precipitation, and the reaction conditions and concentrations during precipitation influence the degree of substitution of phosphate for hydroxyl in the salt. Hydroxyphosphates generally have a PO₄/Al molar ratio between 0.3 and 1.2. Hydroxyphosphates can be distinguished from strict AlPO₄ by the presence of hydroxyl groups. For example, an IR spectrum band at 3164cm⁻¹ (e.g. when heated to 200°C) indicates the presence of structural hydroxyls [ch.9 of ref. 136]

The PO₄/Al³⁺ molar ratio of an aluminum phosphate adjuvant will generally be between 0.3 and 1.2, preferably between 0.8 and 1.2, and more preferably 0.95±0.1. The aluminum phosphate will generally be amorphous, particularly for hydroxyphosphate salts. A typical adjuvant is amorphous aluminum hydroxyphosphate with PO₄/Al molar ratio between 0.84 and 0.92, included at 0.6mg Al³⁺/ml. The aluminum phosphate will generally be particulate (e.g. plate-like morphology as seen in transmission electron micrographs). Typical diameters of the particles are in the range 0.5-20µm (e.g. about 5-10µm) after any antigen adsorption. Adsorptive capacities of between 0.7-1.5 mg protein per mg Al⁺⁺⁺ at pH 7.4 have been reported for aluminum phosphate adjuvants.

The point of zero charge (PZC) of aluminum phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminum phosphates used according to the invention will generally have a PZC of between 4.0 and 7.0, more preferably between 5.0 and 6.5 e.g. about 5.7.

Suspensions of aluminum salts used to prepare compositions of the invention may contain a buffer *(e.g.* a phosphate or a histidine or a Tris buffer), but this is not always necessary. The suspensions are preferably sterile and pyrogen-free. A suspension may include free aqueous phosphate ions e.g. present at a concentration between 1.0 and 20 mM, preferably between 5 and 15 mM, and more preferably about 10 mM. The suspensions may also comprise sodium chloride.

The invention can use a mixture of both an aluminum hydroxide and an aluminum phosphate. In this case there may be more aluminium phosphate than hydroxide e.g. a weight ratio of at least 2:1 *e.g*. ≥5:1, ≥6:1, ≥7:1, ≥8:1, ≥9:1, *etc.*

The concentration of Al⁺⁺⁺ in a composition for administration to a patient is preferably less than 10mg/ml *e.g.* ≤5 mg/ml, ≤4 mg/ml, ≤3 mg/ml, ≤2 mg/ml, ≤1 mg/ml, *etc.* A preferred range is between 0.3 and 1 mg/ml.

### Pharmaceutical compositions

HIV-1 envelope antigens used with the invention will be administered as components in immunogenic compositions in order to elicit an immune response in a patient. The immune response can include a humoral (e.g. an antibody response, such as a neutralizing antibody response) and/or a cellular response. In a patient already infected with HIV, the immune response may reduce the severity of the infection (e.g. reduce viral load) and may even result in clearance of HIV infection (therapeutic immunization). In a patient who is not infected with HIV, the immune response may reduce the risk of future HIV infection and may even be protective against future HIV infection (prophylactic immunization). For therapeutic immunization, the effects arising from administration of the immunogenic composition may be augmented by, or also require, the use of other anti-HIV strategies e.g. the administration of antivirals, including but not limited to nucleoside reverse transcriptase inhibitors, non-nucleoside reverse transcriptase inhibitors, protease inhibitors, entry inhibitors, fusion inhibitors, etc.

Immunogenic compositions will include an immunologically effective amount of a HIV-1 envelope antigen. By 'immunologically effective amount', it is meant that the administration of that amount to an individual, either in a single dose or as part of a series, is effective for the desired treatment or prevention. This amount can vary depending upon the health and physical condition of the individual to be treated, age, the taxonomic group of individual to be treated (e.g. non-human primate, primate, *etc.),* the capacity of the individual's immune system to synthesize antibodies, the degree of protection desired, the formulation of the vaccine, the treating physician's assessment of the medical situation, and other relevant factors. It is expected that the amount will fall in a relatively broad range that can be determined through routine trials, and a typical quantity of antigen per dose is between 1 µg and 1mg e.g. about 100µg per antigen per dose.

Immunogenic compositions of the invention are pharmaceutically acceptable. They usually include components in addition to the complexes e.g. they typically include one or more pharmaceutical carrier(s) and/or excipient(s). A thorough discussion of such components is available in ref. 143.

Compositions will generally be in aqueous form.

To control tonicity, it is preferred to include a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride, calcium chloride, etc.

Compositions, particularly those for parenteral administration, will generally have an osmolality of between 200 mOsm/kg and 400 mOsm/kg, preferably between 240-360 mOsm/kg, and will more preferably fall within the range of 290-310 mOsm/kg.

Compositions may include one or more buffers. Typical buffers include: a phosphate buffer; a Tris buffer; a borate buffer; a succinate buffer; a histidine buffer; or a citrate buffer. Buffers will typically be included in the 5-20mM range.

The pH of a composition will generally be between 5 and 8, and more typically between 6 and 7.

The composition is preferably sterile. The composition is preferably non-pyrogenic e.g. containing <1 EU (endotoxin unit, a standard measure) per dose, and preferably <0.1 EU per dose. The composition is preferably gluten free.

Compositions of the invention may include detergent e.g. a polyoxyethylene sorbitan ester surfactant (known as 'Tweens'), an octoxynol (such as octoxynol-9 (Triton X-100) or t-octylphenoxypolyethoxyethanol), *etc.*

Vaccines for injection may be administered in a dosage volume of about 0.5ml.

Vaccines for intranasal administration may be administered in a dosage volume of about 0.3ml. This volume may be administered in a single nostril or be split between both nostrils.

### Patients

The invention is particularly suitable for use with human patients, but can also be used with other mammals for investigational purposes, for raising antisera, *etc.*

Typical patients will be adolescents (e.g. 12-17 years old) and adults (e.g. 18-55 years old).

### Kits of the invention

Where a composition includes an antigen and an adjuvant, these may be mixed during manufacture, or they may be mixed extemporaneously, at the time of delivery. In some cases, the adjuvant and antigen can be administered to a patient separately, without the need for mixing. Thus a combination of antigen and adjuvant may be provided in the form of a kit including the various components, either ready for mixing at the time of use, or ready for separate administration.

The components are physically separate from each other within the kit, and this separation can be achieved in various ways. For instance, the two components may be in two separate containers, such as vials. The contents of the two vials can then be mixed e.g. by removing the contents of one vial and adding them to the other vial, or by separately removing the contents of both vials and mixing them in a third container.

In a preferred arrangement, one of the kit components is in a syringe and the other is in a container such as a vial. The syringe can be used (e.g. with a needle) to insert its contents into the second container for mixing, and the mixture can then be withdrawn into the syringe. The mixed contents of the syringe can then be administered to a patient, typically through a new sterile needle. Packing one component in a syringe eliminates the need for using a separate syringe for patient administration.

In another preferred arrangement, the two kit components are held together but separately in the same syringe e.g. a dual-chamber syringe, such as those disclosed in references 144-151 *etc.* When the syringe is actuated (e.g. during administration to a patient) then the contents of the two chambers are mixed. This arrangement avoids the need for a separate mixing step at the time of use.

The kit components will generally be in aqueous form. In some arrangements, a component (typically the antigen component rather than the adjuvant component) is in dry form (e.g. in a lyophilized form), with the other component being in aqueous form. The two components can be mixed in order to reactivate the dry component and give an aqueous composition for administration to a patient. A lyophilized component will typically be located within a vial rather than a syringe. Dried components may include stabilizers such as lactose, sucrose or mannitol, as well as mixtures thereof e.g. lactose/sucrose mixtures, sucrose/mannitol mixtures, *etc.* One possible arrangement uses an aqueous adjuvant component in a pre-filled syringe and a lyophilized antigen component in a vial.

### General

The term "comprising" encompasses "including" as well as "consisting" e.g. a composition "comprising" X may consist exclusively of X or may include something additional e.g. X + Y.

The word "substantially" does not exclude "completely" e.g. a composition which is "substantially free" from Y may be completely free from Y. Where necessary, the word "substantially" may be omitted from the definition of the invention.

The term "about" in relation to a numerical value x means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc.*

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

The term "polypeptide" refers to amino acid polymers of any length. The polymer may be linear or branched, it may comprise modified amino acids, and it may be interrupted by non-amino acids. The terms also encompass an amino acid polymer that has been modified naturally or by intervention; for example, disulfide bond formation, glycosylation, lipidation, acetylation, phosphorylation, or any other manipulation or modification, such as conjugation with a labeling component. Also included within the definition are, for example, polypeptides containing one or more analogs of an amino acid (including, for example, unnatural amino acids, *etc.),* as well as other modifications known in the art. Polypeptides can occur as single chains or associated chains. Polypeptides of the invention can be naturally or non-naturally glycosylated (*i.e*. the polypeptide has a glycosylation pattern that differs from the glycosylation pattern found in the corresponding naturally occurring polypeptide).

Polypeptides for use with the invention can be prepared in many ways e.g. by chemical synthesis (in whole or in part), by digesting longer polypeptides using proteases, by translation from RNA, by purification from cell culture (e.g. from recombinant expression), from the organism itself (e.g. after bacterial culture, or direct from patients), *etc.* A preferred method for production of peptides <40 amino acids long involves *in vitro* chemical synthesis [152,153]. Solid-phase peptide synthesis is particularly preferred, such as methods based on tBoc or Fmoc [154] chemistry. Enzymatic synthesis [155] may also be used in part or in full. As an alternative to chemical synthesis, biological synthesis may be used e.g. the polypeptides may be produced by translation. This may be carried out *in vitro* or *in vivo.* Biological methods are in general restricted to the production of polypeptides based on L-amino acids, but manipulation of translation machinery (e.g. of aminoacyl tRNA molecules) can be used to allow the introduction of D-amino acids (or of other non natural amino acids, such as iodotyrosine or methylphenylalanine, azidohomoalanine, etc.) [156]. Where D-amino acids are included, however, it is preferred to use chemical synthesis. Polypeptides of the invention may have covalent modifications at the C-terminus and/or N-terminus.

Polypeptides can take various forms (e.g. native, fusions, glycosylated, non-glycosylated, lipidated, non-lipidated, phosphorylated, non-phosphorylated, myristoylated, non-myristoylated, monomeric, multimeric, particulate, denatured, *etc.).* For HIV-1 envelope antigen, oligomeric glycosylated polypeptides are preferred.

Polypeptides are preferably provided in purified or substantially purified form *i.e*. substantially free from other polypeptides (e.g. free from naturally-occurring polypeptides), particularly from other HIV or host cell polypeptides, and are generally at least about 50% pure (by weight), and usually at least about 90% pure *i.e*. less than about 50%, and more preferably less than about 10% *(e.g.* 5% or less) of a composition is made up of other expressed polypeptides.

### MODES FOR CARRYING OUT THE INVENTION

### Mouse experiments

Five groups of 10 Balb-c mice received the following:
● Groups 1-4 received 3 intranasal immunizations with doses of 0.5 µg, 5µg, 25 µg, or 50 µg of o-gp140ΔV2_{SFI62} protein with 10 µg LTK63.
● Group 5 received 3 intranasal doses of 10 µg LTK63, as a control.

Intranasal doses were administered in a total aqueous volume of 40µl, at two week intervals (0, 2, & 4 weeks). All five groups then received two intramuscular boosters (6 & 8 weeks) with 25µg of o-gp140ΔV2_{SF162} protein in combination with a MF59 adjuvant (50 µl).

Serum anti-Env IgG and vaginal anti-Env IgA were measured after each immunization.

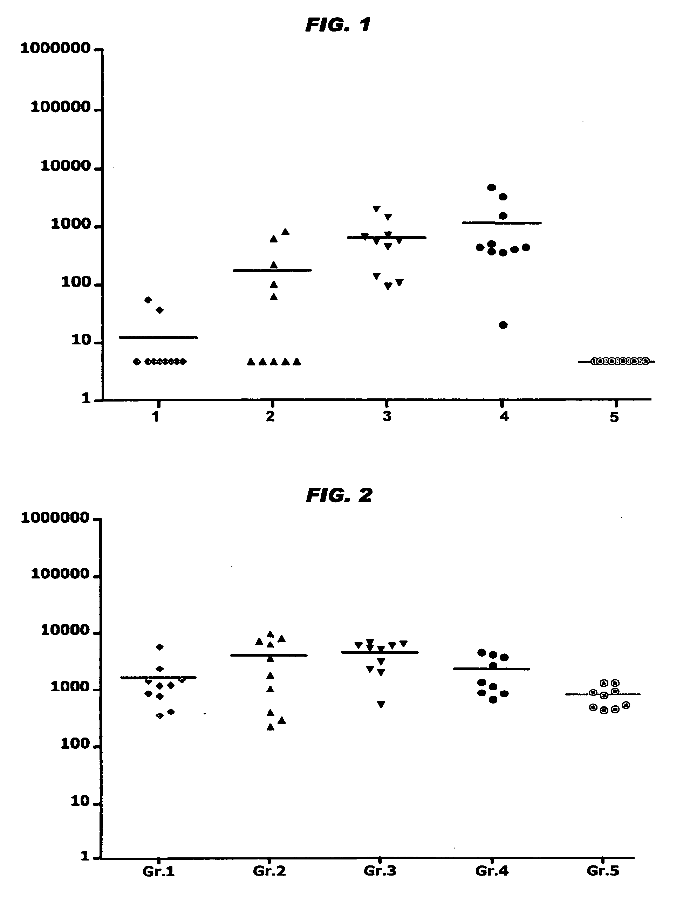

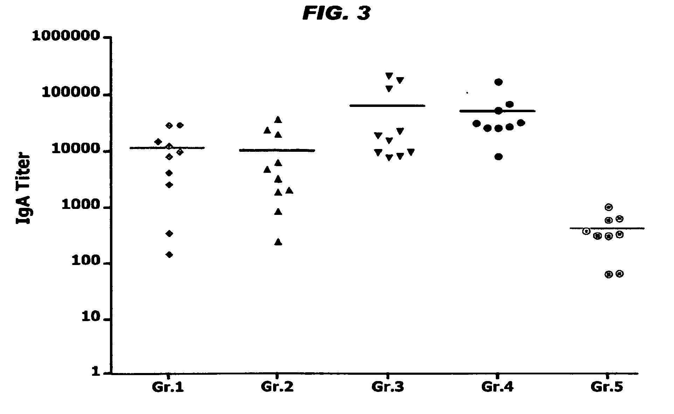

IgG levels 2 weeks after the third intranasal dose, immediately preceding the first intramuscular dose, are shown in Figure 1. IgG levels 2 weeks after the second intramuscular dose are in Figure 2. A comparison of Figures 1 and 2 shows that the intramuscular immunizations increase IgG titers in all groups. Good IgA responses were also seen after the fifth dose (Figure 3). For both IgG and IgA, the highest mean titer was seen in group 3 (25µg antigen).

### Human clinical trial

Three groups of 10 subjects (aged 18-45 years) receive (i) three nasal doses of LT-K63 (30µg per dose), (ii) o-gp 140ΔV2_{SF162} protein (100µg per dose), or (iii) a mixture of both. All patients then receive two intramuscular doses of o-gp140ΔV2_{SF162}(100µg per dose) in a MF59 adjuvant.

For the intranasal doses, the separate components (antigen and/or adjuvant) are mixed before administration to a final volume of 284 µL, and this volume is equally distributed between left and right nostrils via a 200µl pipette.

For the intramuscular doses, a solution of o-gp140ΔV2_{SFI62} is diluted to give 100µg at 2x concentration. This solution is then mixed at a 1:1 volume ratio with MF59, to give a final solution.

The five doses are administered at time 0, 4 weeks, 8 weeks, 16 weeks and 28 weeks.

To assess immunogenicity, blood samples are taken before time 0, at time 0, week 4 minus 1 day, week 8 minus 1 day, then at weeks 10, 12, 16, 28 & 32. Nasal and vaginal (for females) samples are taken at the same times.

Total serum IgG antibody responses against HIV-1 Env are measured in the blood samples using standard indirect ELISA. Similarly, total nasal and vaginal secretory IgA antibody responses against HIV-1 Env are measured using standard indirect ELISA. Moreover, T-cell responses to HIV are measured by assessing interferon-γ release by ELISpot assay of PBMCs in response to stimulation with HIV peptides. Similarly, serum functional antibody responses are measured by assessing virus neutralisation against the homologous HIV-1 strain.

It will be understood that the invention has been described by way of example only and modifications may be made whilst remaining within the scope and spirit of the invention.

Further embodiments of the invention are described below:
1. A method for raising an immune response in a patient, comprising: (i) administering a HIV-1 antigen to the patient via a mucosal route; and then (ii) 25 administering a HIV-1 envelope antigen to the patient via a parenteral route.
2. The use of a HIV-1 envelope antigen in the manufacture of a medicament for administration to a patient by a method comprising: (i) administering the HIV-1 envelope antigen to the patient via a mucosal route; and then (ii) administering a HIV-1 envelope antigen to the patient via a parenteral route.
3. A method for raising an immune response in a patient, comprising: administering a HIV-1 envelope antigen to the patient via a parenteral route, wherein the patient has previously received a HIV-1 envelope antigen via a mucosal route.
4. The use of a HIV-1 envelope antigen in the manufacture of a medicament for administration to a patient who has previously received a HIV-1 envelope antigen via a mucosal route, wherein the medicament is for administration to the patient via a parenteral route.
5. A kit comprising (i) a HIV-1 envelope antigen for administration to a patient via a mucosal route; and (ii) a HIV-1 envelope antigen for administration to a patient via a parenteral route.
6. The method, use or kit of embodiments 1-5, wherein the HIV-1 envelope antigens are adjuvanted.
7. The method, use or kit of embodiments 1-6, wherein mucosal immunizations are via the intranasal route.
8. The method, use or kit of embodiments 1-7, wherein parenteral immunizations are via the intramuscular route.
9. The method, use or kit of embodiments 1-8, wherein mucosal immunizations include a detoxified mutant *ofE.coli* heat labile toxin as adjuvant.
10. The method, use or kit of embodiments 1-9, wherein parenteral immunizations include an oil-in-water emulsion as adjuvant.
11. The method, use or kit of embodiments 1-10, wherein more than one mucosal immunization is administered.
12. The method, use or kit of embodiments 1-11, wherein more than one parenteral immunization is administered.
13. The method, use or kit of claims 1-12, wherein the mucosal immunization is intranasal.
14. The method, use or kit of claims 1-13, wherein the parenteral immunization is intramuscular.
15. The method, use or kit of embodiments 1-4, wherein each immunization uses the same HIV-1 envelope antigen.
16. The method, use or kit of embodiments 1-13, wherein each immunization uses a different HIV-1 envelope antigen.
17. The method, use or kit of embodiments 15-16, wherein the envelope antigen is o-gp140ΔV2_{SF162}.
18. The method, use or kit of embodiments 1-17, wherein the mucosal immunization has three intranasal doses of a composition with between 10µg and 50µg of LT-K63 adjuvant per dose.
19. The method, use or kit of embodiments 1-18, wherein the parenteral immunization has two intramuscular doses of a composition with a squalene-in-water emulsion.
20. The method, use or kit of embodiments 1-19, wherein the patient is a human aged between 12 and 55 years old.
21. The method, use or kit of embodiments 1-20, wherein the patient is not infected with HIV-1.
22. The method, use or kit of embodiments 1-20, wherein the patient is infected with HIV-1.
23. A HIV-1 envelope antigen in combination with a mucosal adjuvant, and a HIV-1 envelope antigen in combination with a parenteral adjuvant, for simultaneous separate or sequential administration.
24. An immunogenic composition for intranasal administration, comprising a HIV-1 envelope antigen in combination with a detoxified mutant *E. coli* heat labile toxin ('LT'), wherein a unit dose of the composition includes between 10µg and 1mg of Env antigen and between 10µg and 50µg of LT.
25. An immunogenic composition for intranasal administration, comprising a HIV-1 envelope antigen in combination with a detoxified mutant of *E. coli* heat labile toxin ('LT'), wherein the weight ratio of Env antigen to LT is between 2:1 and 4:1.
26. An immunogenic composition for intramuscular administration, comprising a HIV-1 envelope antigen in combination with an oil-in-water emulsion adjuvant, wherein a unit dose of the composition includes between 10µg and 1mg of envelope antigen.

### REFERENCES (the contents of which are hereby incorporated by reference)

[1] WO 03/059385.
[2] WO 02/080648.
[3] Robertson el al. (2000) Science 288:55-56.
[4] *http:*//*hiv-lveb.lanl.gov*/
[5] WO 00/39302.
[6] WO 03/020876.
[7] WO 2005/007808.
[8] WO 03/004620.
[9] WO 00/39304.
[10] U.S. Patent No. 5,792,459.
[11] Zhang et al. (2001) J. Biol. Chem. 276:39577-39585.
[12] Earl et al. (2001) J. Virol. 75:645-653.
[13] Barnett et al. (2001) J. Virol. 75:5526-5540.
[14] Srivastava et al. (2003) J. Virol. 77:2310-2320.
[15] WO 91/15238.
[16] Geysen et al. (1984) Proc. Natl. Acad. Sci. USA 81:3998-4002.
[17] Carter (1994) Methods Mol. Biol. 36:207-223.
[18] Jameson, BA et al. 1988, CABIOS 4(1):181-186.
[19] Raddrizzani & Hammer (2000) BriefBioinform. 1(2):179-189.
[20] De Lalla et al. (1999) J. Immunol. 163:1725-1729.
[21] Brusic et al. (1998) Bioinformatics 14(2):121-130
[22] Meister et al. (1995) Vaccine 13(6):581-591.
[23] Roberts et al. (1996) AIDS Res. Hum. Retroviruses 12(7):593-610.
[24] Maksyutov & Zagrebelnaya (1993) Comput Appl. Biosci. 9(3):291-297.
[25] Feller & de la Cruz (1991) Nature 349(6311):720-721.
[26] Hopp (1993) Peptide Research 6:183-190.
[27] Welling et al. (1985) FEBS Lett. 188:215-218.
[28] Davenport et al. (1995) Immunogenetics 42:392-397.
[29] Needleman& Wunsch (1970) J. Mol. Biol. 48:443-453.
[30] Rice et al. (2000) Trends Genet. 16:276-277.
[31] Ensoli et al. (2005) Microbes Infect. 7:1392-1399.
[32] WO 2005/090391.
[33] Greenbaum et al. (2004) Vaccine 22:2566-2577.
[34] Zurbriggen et al. (2003) Expert Rev. Vaccines 2:295-304*.*
[35] Piascik (2003) J. Am. Pharm. Assoc. (Wash DC). 43:728-730.
[36] Mann et al. (2004) Vaccine 22:2425-2429.
[37] Almeida & Alpar (1996) J. Drug Targeting 3:455-467.
[38] Agarwal & Mishra (1999) Indian J. Exp. Biol. 37:6-16.
[39] Michetti (1998) J Gastroenterol. [Suppl X]:66-68.
[40] Pizza et al. (2000) Int. J. Med. Microbiol. 290:455-461.
[41] WO 95/17211.
[42] WO 99/52549.
[43] WO 01/21207.
[44] WO 01/21152.
[45] WO 00/50078.
[46] Singh et al. (2001) J. Cont. Release 70:267-276.
[47] WO 99/27960.
[48] WO 99/27960.
[49] WO00/62800.
[50] U.S. Patent No. 6,090,406
[51] US 5,916,588.
[52] EP-A-0626169.
[53] Johnson et al. (1999) Bioorg. Med. Chem. Lett. 9:2273-2278.
[54] Evans et al. (2003) Expert Rev. Vaccines 2:219-229.
[55] Andrianov et al. (1998) Biomaterials 19:109-115.
[56] Payne et al. (1998) Adv. Drug Delivery Review 31:185-196.
[57] Burdman et al. (eds.) (1995) Vaccine Design: The Subunit and Adjuvant Approach, 1st edition (Springer). ISBN: 030644867X
[58] WO 98/18928.
[59] Del Giudice et al. (1998) Molecular Aspects of Medicine, vol. 19, number 1.
[60] Park et al. (2000) Exp. Mol. Med. 32:72-78.
[61] Domenighini et al. (1995) Mol. Microbiol. 15:1165-1167.
[62] WO 01/22972.
[63] Kandimalla et al. (2003) Nucleic Acids Research 31:2393-2400.
[64] WO 02/26757.
[65] WO 99/62923.
[66] Krieg (2003) Nature Medicine 9:831-835.
[67] McCluskie et al. (2002) FEMS Immunol. Med. Microbiol. 32:179-185.
[68] WO 98/40100.
[69] U.S. Patent No. 6,207,646.
[70] U.S. Patent No. 6,239,116.
[71] U.S. Patent No. 6,429,199.
[72] Kandimalla et al. (2003) Biochem. Soc. Trans. 31 (part 3):654-658.
[73] Blackwell et al. (2003) J. Immunol. 170:4061-4068.
[74] Krieg (2002) Trends Immunol. 23:64-65.
[75] WO 01/95935.
[76] Kandimalla et al. (2003) BBRC 306:948-953.
[77] Bhagat et al. (2003) BBRC 300:853-861.
[78] WO 03/035836.
[79] Myers et al. (1990) pages 145-156 of Cellular and molecular aspects of endotoxin reactions*.*
[80] Ulrich (2000) Chapter 16 (pages 273-282) of reference 137.
[81] Johnson et al. (1999) J. Med. Chem. 42:4640-4649.
[82] Baldrick et al. (2002) Regulatory Toxicol. Pharmacol. 35:398-413.
[83] UK patent application GB-A-2220211.
[84] Wheeler et al. (2001) International Archives of Allergy and Immunology 126:135-139
[85] U.S. Patent No. 4,680,338.
[86] U.S. Patent No. 4,988,815.
[87] WO 92/15582.
[88] Stanley (2002) Clin. Exp. Dermatol. 27:571-577.
[89] Wu et al. (2004) Antiviral Res. 64(2):79-83.
[90] Vasilakos et al. (2000) Cell. Immunol.. 204(1):64-74.
[91] U.S. Patent Nos. 4,689,338; 4,929,624; 5,238,944; 5,266,575; 5,268,376; 5,346,905; 5,352,784; 5,389,640; 5,395,937; 5,482,936; 5,494,916; 5,525,612; 6,083,505; 6,440,992; 6,627,640; 6,656,938; 6,660,735; 6,660,747; 6,664,260; 6,664,264; 6,664,265; 6,667,312; 6,670,372; 6,677,347; 6,677,348; 6,677,349; 6,683,088; 6,703,402; 6,743,920; 6,800,624; 6,809,203; 6,888,000; and 6,924,293.
[92] Jones (2003) Curr. Opin. Investig. Drugs 4:214-218.
[93] WO 2004/060308.
[94] WO 2004/064759.
[95] U.S. Patent No. 6,355,271.
[96] WO 00/23105.
[97] U.S. Patent No. 6,924,271.
[98] U.S. Publication No. 2005/0070556.
[99] U.S. Patent No. 5,658,731.
[100] U.S. Patent No. 5,011,828.
[101] WO 2004/87153.
[102] U.S. Patent No. 6,605,617.
[103] WO 02/18383.
[104] WO 2004/018455.
[105] WO 03/082272.
[106] WO 2006/002422.
[107] Thompson et al. (2005) Journal of Leukocyte Biology 78:1273-1280
[108] U.S. Patent No. 5,057,540.
[109] WO 96/33739.
[110] EP-A-0109942.
[111] WO 96/11711.
[112] WO 00/07621.
[113] Barr et al. (1998) Advanced Drug Delivery Reviews 32:247-271.
[114] Sjolanderet et al. (1998) Advanced Drug Delivery Reviews 32:321-338.
[115] WO 98/42375.
[116] Feng et al. (2005) Acta Biochim Biophys Sin (Shanghai). 37(2):126-32.
[117] Barackman et al. (1999) Infect Immun 67(8):4276-9.
[118] Singh et al. (1998) Vaccine 16(19):1822-7.
[119] Rosenkrands et al. (2005) Infect Immun 73(9):5817-26.
[120] US patent 6355271.
[121] He et al. (2000) Clin Diagn Lab Immunol 7(6): 899-903.
[122] Signorelli & Hadden (2003) Int. Immunopharmacol. 3(8):1177-1186.
[123] WO 2004/064715.
[124] Cooper (1995) Pharm. Biotechnol. 6:559-580.
[125] Silva et al. (2004) Immunol Cell Biol 82(6):611-6*.*
[126] Han et al. (2004) Ann N YAcad Sci 1031:96-101.
[127] WO 03/011223.
[128] Meraldi et al. (2003) Vaccine 21:2485-2491.
[129] Pajak et al. (2003) Vaccine 21:836-842.
[130] U.S. Patent No. 6,586,409.
[131] Wrong et al. (2003) J. Clin. Pharmacol. 43(7):735-742.
[132] U.S. Publication No. 2005/0215517.
[133] WO 90/14837.
[134] Podda & Del Giudice (2003) Expert Rev. Vaccines 2:197-203.
[135] Podda (2001) Vaccine 19: 2673-2680.
[136]Burdman et al. (eds.) (1995) Vaccine Design: The Subunit and Adjuvant Approach, 1st edition (Springer). ISBN: 030644867X.
[137]O'Hagan (ed.) (2000) Vaccine Adjuvants: Preparation Methods and Research Protocols (Volume 42 of Methods in Molecular Medicine series) (Humana Press). ISBN: 0896037355.
[138] Allison & Byars (1992) Res. Immunol. 143:519-525.
[139] Hariharan et al. (1995) Cancer Res. 55:3486-3489.
[140] WO 95/11700.
[141] U.S. Patent No. 6,080,725.
[142] WO 2005/097181.
[143] Gennaro (2000) Remington: The Science and Practice of Pharmacy, 20th edition (Lippincott Williams & Wilkens). ISBN: 0683306472.
[144] WO 2005/089837.
[145] U.S. Patent No. 6,692,468.
[146] WO 00/07647.
[147] WO 99/17820.
[148] U.S. Patent No. 5,971,953.
[149] U.S. Patent No. 4,060,082.
[150] EP-A-0520618.
[151] WO 98/01174.
[152]Bodanszky (1993) Principles of Peptide Synthesis, 2nd edition (Springer-Verlag). ISBN: 0387564314.
[153]Fields et al. (1997) Methods In Enzymology, Volume 289: Solid-Phase Peptide Synthesis, 1st edition (Academic Press). ISBN: 0121821900.
[154]Chan & White (2000) Fmoc Solid Phase Peptide Synthesis (Oxford University Press). ISBN: 0199637245.
[155] Kullmann (1987) Enzymatic Peptide Synthesis. ISBN: 0849368413.
[156] Ibba (1996) Biotechnol. Genet. Eng. Rev. 13:197-216.

## Claims

1. An HIV-1 envelope antigen, for use in a method for raising an immune response in a patient, comprising: (i) administering at least one dose of a HIV-1 envelope antigen to the patient via a mucosal route; and then (ii) administering at least one of a HIV-1 envelope antigen to the patient via a parenteral route.

2. The use of a HIV-1 envelope antigen in the manufacture of a medicament for administration to a patient by a method comprising: (i) administering at least one dose of the HIV-1 envelope antigen to the patient via a mucosal route; and then (ii) administering at least one dose of a HIV-1 envelope antigen to the patient via a parenteral route.

3. An HIV-1 envelope antigen, for use in a method for raising an immune response in a patient, comprising: administering at least one dose of a HIV-1 envelope antigen to the patient via a parenteral route, wherein the patient has previously received at least one dose of a HIV-1 envelope antigen via a mucosal route.

4. The use of a HIV-1 envelope antigen in the manufacture of a medicament for administration of at least one dose to a patient who has previously received at least one dose of a HIV-1 envelope antigen via a mucosal route, wherein the medicament is for administration to the patient via a parenteral route.

5. A kit comprising (i) at least one dose of a HIV-1 envelope antigen for administration to a patient via a mucosal route; and (ii) at least one dose of a HIV-1 envelope antigen for administration to a patient via a parenteral route, following administration of the first at least one dose via a mucosal route.

6. The antigen, use or kit of claims 1-5, wherein the HIV-1 envelope antigens are adjuvanted.

7. The antigen, use or kit of claims 1-6, wherein mucosal immunizations include a detoxified mutant of *E. coli* heat labile toxin as adjuvant.

8. The antigen, use or kit of claims 1-7, wherein parenteral immunizations include an oil-in-water emulsion as adjuvant.

9. The antigen, use or kit of claims 1-8, wherein the mucosal immunization is intranasal.

10. The antigen, use or kit of claims 1-9, wherein the parenteral immunization is intramuscular.

11. The antigen, use or kit of claims 1-10, wherein each immunization uses the same HIV-1 envelope antigen, or wherein each immunization uses a different HIV-1 envelope antigen.

12. The antigen, use or kit of claim 11, wherein the envelope antigen is o-gp140ΔV2_{SF162}.

13. The antigen, use or kit of claims 1-12, wherein the mucosal immunization has three intranasal doses of a composition with between 10µg and 50µg of LT-K63 adjuvant per dose.

14. The antigen, use or kit of claims 1-13, wherein the parenteral immunization has two intramuscular doses of a composition with a squalene-in-water emulsion.

15. The antigen, use or kit of claims 1-14, wherein the patient is (a) a human aged between 12 and 55 years old; (b) not infected with HIV-1; or (c) infected with HIV-1.


SEQUENCE LISTING

<110>   NOVARTIS AG

<120>   COMBINED MUCOSAL AND PARENTERAL IMMUNIZATION AGAINST HIV

<130>   P056618EP

<150>   US 60/788,563
<151>   2006-03-31

<150>   US 60/840,178
<151>   2006-08-26

<150>   PCT/US2007/007583
<151>   2007-03-29

<150>   EP 07773929.0
<151>   2007-03-29

<160>   6

<170>   SeqWin99, version 1.02

<210>   1
<211>   856
<212>   PRT
<213>   Human immunodeficiency virus HIV-1

<400>   1
Met Arg Val Lys Glu Lys Tyr Gln His Leu Trp Arg Trp Gly Trp Arg
1               5                   10                  15

Trp Gly Thr Met Leu Leu Gly Met Leu Met Ile Cys Ser Ala Thr Glu
            20                  25                  30

Lys Leu Trp Val Thr Val Tyr Tyr Gly Val Pro Val Trp Lys Glu Ala
        35                  40                  45

Thr Thr Thr Leu Phe Cys Ala Ser Asp Ala Lys Ala Tyr Asp Thr Glu
    50                  55                  60

Val His Asn Val Trp Ala Thr His Ala Cys Val Pro Thr Asp Pro Asn
65                  70                  75                  80

Pro Gln Glu Val Val Leu Val Asn Val Thr Glu Asn Phe Asn Met Trp
                85                  90                  95

Lys Asn Asp Met Val Glu Gln Met His Glu Asp Ile Ile Ser Leu Trp
            100                 105                 110

Asp Gln Ser Leu Lys Pro Cys Val Lys Leu Thr Pro Leu Cys Val Ser
        115                 120                 125

Leu Lys Cys Thr Asp Leu Lys Asn Asp Thr Asn Thr Asn Ser Ser Ser
    130                 135                 140

Gly Arg Met Ile Met Glu Lys Gly Glu Ile Lys Asn Cys Ser Phe Asn
145                 150                 155                 160

Ile Ser Thr Ser Ile Arg Gly Lys Val Gln Lys Glu Tyr Ala Phe Phe

165                 170                 175

Tyr Lys Leu Asp Ile Ile Pro Ile Asp Asn Asp Thr Thr Ser Tyr Lys
            180                 185                 190

Leu Thr Ser Cys Asn Thr Ser Val Ile Thr Gln Ala Cys Pro Lys Val
        195                 200                 205

Ser Phe Glu Pro Ile Pro Ile His Tyr Cys Ala Pro Ala Gly Phe Ala
    210                 215                 220

Ile Leu Lys Cys Asn Asn Lys Thr Phe Asn Gly Thr Gly Pro Cys Thr
225                 230                 235                 240

Asn Val Ser Thr Val Gln Cys Thr His Gly Ile Arg Pro Val Val Ser
                245                 250                 255

Thr Gln Leu Leu Leu Asn Gly Ser Leu Ala Glu Glu Glu Val Val Ile
            260                 265                 270

Arg Ser Val Asn Phe Thr Asp Asn Ala Lys Thr Ile Ile Val Gln Leu
        275                 280                 285

Asn Thr Ser Val Glu Ile Asn Cys Thr Arg Pro Asn Asn Asn Thr Arg
    290                 295                 300

Lys Arg Ile Arg Ile Gln Arg Gly Pro Gly Arg Ala Phe Val Thr Ile
305                 310                 315                 320

Gly Lys Ile Gly Asn Met Arg Gln Ala His Cys Asn Ile Ser Arg Ala
                325                 330                 335

Lys Trp Asn Asn Thr Leu Lys Gln Ile Ala Ser Lys Leu Arg Glu Gln
            340                 345                 350

Phe Gly Asn Asn Lys Thr Ile Ile Phe Lys Gln Ser Ser Gly Gly Asp
        355                 360                 365

Pro Glu Ile Val Thr His Ser Phe Asn Cys Gly Gly Glu Phe Phe Tyr
    370                 375                 380

Cys Asn Ser Thr Gln Leu Phe Asn Ser Thr Trp Phe Asn Ser Thr Trp
385                 390                 395                 400

Ser Thr Glu Gly Ser Asn Asn Thr Glu Gly Ser Asp Thr Ile Thr Leu
                405                 410                 415

Pro Cys Arg Ile Lys Gln Ile Ile Asn Met Trp Gln Lys Val Gly Lys
            420                 425                 430

Ala Met Tyr Ala Pro Pro Ile Ser Gly Gln Ile Arg Cys Ser Ser Asn
        435                 440                 445

Ile Thr Gly Leu Leu Leu Thr Arg Asp Gly Gly Asn Ser Asn Asn Glu
    450                 455                 460

Ser Glu Ile Phe Arg Pro Gly Gly Gly Asp Met Arg Asp Asn Trp Arg
465                 470                 475                 480

Ser Glu Leu Tyr Lys Tyr Lys Val Val Lys Ile Glu Pro Leu Gly Val
                485                 490                 495

Ala Pro Thr Lys Ala Lys Arg Arg Val Val Gln Arg Glu Lys Arg Ala
            500                 505                 510

Val Gly Ile Gly Ala Leu Phe Leu Gly Phe Leu Gly Ala Ala Gly Ser
        515                 520                 525

Thr Met Gly Ala Ala Ser Met Thr Leu Thr Val Gln Ala Arg Gln Leu
    530                 535                 540

Leu Ser Gly Ile Val Gln Gln Gln Asn Asn Leu Leu Arg Ala Ile Glu
545                 550                 555                 560

Ala Gln Gln His Leu Leu Gln Leu Thr Val Trp Gly Ile Lys Gln Leu
                565                 570                 575

Gln Ala Arg Ile Leu Ala Val Glu Arg Tyr Leu Lys Asp Gln Gln Leu
            580                 585                 590

Leu Gly Ile Trp Gly Cys Ser Gly Lys Leu Ile Cys Thr Thr Ala Val
        595                 600                 605

Pro Trp Asn Ala Ser Trp Ser Asn Lys Ser Leu Glu Gln Ile Trp Asn
    610                 615                 620

His Thr Thr Trp Met Glu Trp Asp Arg Glu Ile Asn Asn Tyr Thr Ser
625                 630                 635                 640

Leu Ile His Ser Leu Ile Glu Glu Ser Gln Asn Gln Gln Glu Lys Asn
                645                 650                 655

Glu Gln Glu Leu Leu Glu Leu Asp Lys Trp Ala Ser Leu Trp Asn Trp
            660                 665                 670

Phe Asn Ile Thr Asn Trp Leu Trp Tyr Ile Lys Leu Phe Ile Met Ile
        675                 680                 685

Val Gly Gly Leu Val Gly Leu Arg Ile Val Phe Ala Val Leu Ser Ile
    690                 695                 700

Val Asn Arg Val Arg Gln Gly Tyr Ser Pro Leu Ser Phe Gln Thr His
705                 710                 715                 720

Leu Pro Thr Pro Arg Gly Pro Asp Arg Pro Glu Gly Ile Glu Glu Glu
                725                 730                 735

Gly Gly Glu Arg Asp Arg Asp Arg Ser Ile Arg Leu Val Asn Gly Ser
            740                 745                 750

Leu Ala Leu Ile Trp Asp Asp Leu Arg Ser Leu Cys Leu Phe Ser Tyr
        755                 760                 765

His Arg Leu Arg Asp Leu Leu Leu Ile Val Thr Arg Ile Val Glu Leu
    770                 775                 780

Leu Gly Arg Arg Gly Trp Glu Ala Leu Lys Tyr Trp Trp Asn Leu Leu
785                 790                 795                 800

Gln Tyr Trp Ser Gln Glu Leu Lys Asn Ser Ala Val Ser Leu Leu Asn
                805                 810                 815

Ala Thr Ala Ile Ala Val Ala Glu Gly Thr Asp Arg Val Ile Glu Val
            820                 825                 830

Val Gln Gly Ala Cys Arg Ala Ile Arg His Ile Pro Arg Arg Ile Arg
        835                 840                 845

Gln Gly Leu Glu Arg Ile Leu Leu
    850                 855

<210>   2
<211>   483
<212>   PRT
<213>   Human immunodeficiency virus HIV-1

<400>   2
Ser Ala Thr Glu Lys Leu Trp Val Thr Val Tyr Tyr Gly Val Pro Val
1               5                   10                  15

Trp Lys Glu Ala Thr Thr Thr Leu Phe Cys Ala Ser Asp Ala Lys Ala
            20                  25                  30

Tyr Asp Thr Glu Val His Asn Val Trp Ala Thr His Ala Cys Val Pro
        35                  40                  45

Thr Asp Pro Asn Pro Gln Glu Val Val Leu Val Asn Val Thr Glu Asn
    50                  55                  60

Phe Asn Met Trp Lys Asn Asp Met Val Glu Gln Met His Glu Asp Ile
65                  70                  75                  80

Ile Ser Leu Trp Asp Gln Ser Leu Lys Pro Cys Val Lys Leu Thr Pro
                85                  90                  95

Leu Cys Val Ser Leu Lys Cys Thr Asp Leu Lys Asn Asp Thr Asn Thr
            100                 105                 110

Asn Ser Ser Ser Gly Arg Met Ile Met Glu Lys Gly Glu Ile Lys Asn
        115                 120                 125

Cys Ser Phe Asn Ile Ser Thr Ser Ile Arg Gly Lys Val Gln Lys Glu
    130                 135                 140

Tyr Ala Phe Phe Tyr Lys Leu Asp Ile Ile Pro Ile Asp Asn Asp Thr
145                 150                 155                 160

Thr Ser Tyr Lys Leu Thr Ser Cys Asn Thr Ser Val Ile Thr Gln Ala
                165                 170                 175

Cys Pro Lys Val Ser Phe Glu Pro Ile Pro Ile His Tyr Cys Ala Pro
            180                 185                 190

Ala Gly Phe Ala Ile Leu Lys Cys Asn Asn Lys Thr Phe Asn Gly Thr
        195                 200                 205

Gly Pro Cys Thr Asn Val Ser Thr Val Gln Cys Thr His Gly Ile Arg
    210                 215                 220

Pro Val Val Ser Thr Gln Leu Leu Leu Asn Gly Ser Leu Ala Glu Glu
225                 230                 235                 240

Glu Val Val Ile Arg Ser Val Asn Phe Thr Asp Asn Ala Lys Thr Ile

245                 250                 255

Ile Val Gln Leu Asn Thr Ser Val Glu Ile Asn Cys Thr Arg Pro Asn
            260                 265                 270

Asn Asn Thr Arg Lys Arg Ile Arg Ile Gln Arg Gly Pro Gly Arg Ala
        275                 280                 285

Phe Val Thr Ile Gly Lys Ile Gly Asn Met Arg Gln Ala His Cys Asn
    290                 295                 300

Ile Ser Arg Ala Lys Trp Asn Asn Thr Leu Lys Gln Ile Ala Ser Lys
305                 310                 315                 320

Leu Arg Glu Gln Phe Gly Asn Asn Lys Thr Ile Ile Phe Lys Gln Ser
                325                 330                 335

Ser Gly Gly Asp Pro Glu Ile Val Thr His Ser Phe Asn Cys Gly Gly
            340                 345                 350

Glu Phe Phe Tyr Cys Asn Ser Thr Gln Leu Phe Asn Ser Thr Trp Phe
        355                 360                 365

Asn Ser Thr Trp Ser Thr Glu Gly Ser Asn Asn Thr Glu Gly Ser Asp
    370                 375                 380

Thr Ile Thr Leu Pro Cys Arg Ile Lys Gln Ile Ile Asn Met Trp Gln
385                 390                 395                 400

Lys Val Gly Lys Ala Met Tyr Ala Pro Pro Ile Ser Gly Gln Ile Arg
                405                 410                 415

Cys Ser Ser Asn Ile Thr Gly Leu Leu Leu Thr Arg Asp Gly Gly Asn
            420                 425                 430

Ser Asn Asn Glu Ser Glu Ile Phe Arg Pro Gly Gly Gly Asp Met Arg
        435                 440                 445

Asp Asn Trp Arg Ser Glu Leu Tyr Lys Tyr Lys Val Val Lys Ile Glu
    450                 455                 460

Pro Leu Gly Val Ala Pro Thr Lys Ala Lys Arg Arg Val Val Gln Arg
465                 470                 475                 480

Glu Lys Arg


<210>  3
<211>  345
<212>  PRT
<213>  Human immunodeficiency virus HIV-1

<400>  3
Ala Val Gly Ile Gly Ala Leu Phe Leu Gly Phe Leu Gly Ala Ala Gly
1               5                   10                  15

Ser Thr Met Gly Ala Ala Ser Met Thr Leu Thr Val Gln Ala Arg Gln
            20                  25                  30

Leu Leu Ser Gly Ile Val Gln Gln Gln Asn Asn Leu Leu Arg Ala Ile
        35                  40                  45

Glu Ala Gln Gln His Leu Leu Gln Leu Thr Val Trp Gly Ile Lys Gln
    50                  55                  60

Leu Gln Ala Arg Ile Leu Ala Val Glu Arg Tyr Leu Lys Asp Gln Gln
65                  70                  75                  80

Leu Leu Gly Ile Trp Gly Cys Ser Gly Lys Leu Ile Cys Thr Thr Ala
                85                  90                  95

Val Pro Trp Asn Ala Ser Trp Ser Asn Lys Ser Leu Glu Gln Ile Trp
            100                 105                 110

Asn His Thr Thr Trp Met Glu Trp Asp Arg Glu Ile Asn Asn Tyr Thr
        115                 120                 125

Ser Leu Ile His Ser Leu Ile Glu Glu Ser Gln Asn Gln Gln Glu Lys
    130                 135                 140

Asn Glu Gln Glu Leu Leu Glu Leu Asp Lys Trp Ala Ser Leu Trp Asn
145                 150                 155                 160

Trp Phe Asn Ile Thr Asn Trp Leu Trp Tyr Ile Lys Leu Phe Ile Met
                165                 170                 175

Ile Val Gly Gly Leu Val Gly Leu Arg Ile Val Phe Ala Val Leu Ser
            180                 185                 190

Ile Val Asn Arg Val Arg Gln Gly Tyr Ser Pro Leu Ser Phe Gln Thr
        195                 200                 205

His Leu Pro Thr Pro Arg Gly Pro Asp Arg Pro Glu Gly Ile Glu Glu
    210                 215                 220

Glu Gly Gly Glu Arg Asp Arg Asp Arg Ser Ile Arg Leu Val Asn Gly
225                 230                 235                 240

Ser Leu Ala Leu Ile Trp Asp Asp Leu Arg Ser Leu Cys Leu Phe Ser
                245                 250                 255

Tyr His Arg Leu Arg Asp Leu Leu Leu Ile Val Thr Arg Ile Val Glu
            260                 265                 270

Leu Leu Gly Arg Arg Gly Trp Glu Ala Leu Lys Tyr Trp Trp Asn Leu
        275                 280                 285

Leu Gln Tyr Trp Ser Gln Glu Leu Lys Asn Ser Ala Val Ser Leu Leu
    290                 295                 300

Asn Ala Thr Ala Ile Ala Val Ala Glu Gly Thr Asp Arg Val Ile Glu
305                 310                 315                 320

Val Val Gln Gly Ala Cys Arg Ala Ile Arg His Ile Pro Arg Arg Ile
                325                 330                 335

Arg Gln Gly Leu Glu Arg Ile Leu Leu
            340                 345

<210>   4
<211>   637
<212>   PRT

<213>   Human immunodeficiency virus HIV-1

<400>   4
Ser Ala Thr Glu Lys Leu Trp Val Thr Val Tyr Tyr Gly Val Pro Val
1               5                   10                  15

Trp Lys Glu Ala Thr Thr Thr Leu Phe Cys Ala Ser Asp Ala Lys Ala
            20                  25                  30

Tyr Asp Thr Glu Val His Asn Val Trp Ala Thr His Ala Cys Val Pro
        35                  40                  45

Thr Asp Pro Asn Pro Gln Glu Val Val Leu Val Asn Val Thr Glu Asn
    50                  55                  60

Phe Asn Met Trp Lys Asn Asp Met Val Glu Gln Met His Glu Asp Ile
65                  70                  75                  80

Ile Ser Leu Trp Asp Gln Ser Leu Lys Pro Cys Val Lys Leu Thr Pro
                85                  90                  95

Leu Cys Val Ser Leu Lys Cys Thr Asp Leu Lys Asn Asp Thr Asn Thr
            100                 105                 110

Asn Ser Ser Ser Gly Arg Met Ile Met Glu Lys Gly Glu Ile Lys Asn
        115                 120                 125

Cys Ser Phe Asn Ile Ser Thr Ser Ile Arg Gly Lys Val Gln Lys Glu
    130                 135                 140

Tyr Ala Phe Phe Tyr Lys Leu Asp Ile Ile Pro Ile Asp Asn Asp Thr
145                 150                 155                 160

Thr Ser Tyr Lys Leu Thr Ser Cys Asn Thr Ser Val Ile Thr Gln Ala
                165                 170                 175

Cys Pro Lys Val Ser Phe Glu Pro Ile Pro Ile His Tyr Cys Ala Pro
            180                 185                 190

Ala Gly Phe Ala Ile Leu Lys Cys Asn Asn Lys Thr Phe Asn Gly Thr
        195                 200                 205

Gly Pro Cys Thr Asn Val Ser Thr Val Gln Cys Thr His Gly Ile Arg
    210                 215                 220

Pro Val Val Ser Thr Gln Leu Leu Leu Asn Gly Ser Leu Ala Glu Glu
225                 230                 235                 240

Glu Val Val Ile Arg Ser Val Asn Phe Thr Asp Asn Ala Lys Thr Ile
                245                 250                 255

Ile Val Gln Leu Asn Thr Ser Val Glu Ile Asn Cys Thr Arg Pro Asn
            260                 265                 270

Asn Asn Thr Arg Lys Arg Ile Arg Ile Gln Arg Gly Pro Gly Arg Ala
        275                 280                 285

Phe Val Thr Ile Gly Lys Ile Gly Asn Met Arg Gln Ala His Cys Asn
    290                 295                 300

Ile Ser Arg Ala Lys Trp Asn Asn Thr Leu Lys Gln Ile Ala Ser Lys

305                 310                 315                 320

Leu Arg Glu Gln Phe Gly Asn Asn Lys Thr Ile Ile Phe Lys Gln Ser
                325                 330                 335

Ser Gly Gly Asp Pro Glu Ile Val Thr His Ser Phe Asn Cys Gly Gly
            340                 345                 350

Glu Phe Phe Tyr Cys Asn Ser Thr Gln Leu Phe Asn Ser Thr Trp Phe
        355                 360                 365

Asn Ser Thr Trp Ser Thr Glu Gly Ser Asn Asn Thr Glu Gly Ser Asp
    370                 375                 380

Thr Ile Thr Leu Pro Cys Arg Ile Lys Gln Ile Ile Asn Met Trp Gln
385                 390                 395                 400

Lys Val Gly Lys Ala Met Tyr Ala Pro Pro Ile Ser Gly Gln Ile Arg
                405                 410                 415

Cys Ser Ser Asn Ile Thr Gly Leu Leu Leu Thr Arg Asp Gly Gly Asn
            420                 425                 430

Ser Asn Asn Glu Ser Glu Ile Phe Arg Pro Gly Gly Gly Asp Met Arg
        435                 440                 445

Asp Asn Trp Arg Ser Glu Leu Tyr Lys Tyr Lys Val Val Lys Ile Glu
    450                 455                 460

Pro Leu Gly Val Ala Pro Thr Lys Ala Lys Arg Arg Val Val Gln Arg
465                 470                 475                 480

Glu Lys Arg Ala Val Gly Ile Gly Ala Leu Phe Leu Gly Phe Leu Gly
                485                 490                 495

Ala Ala Gly Ser Thr Met Gly Ala Ala Ser Met Thr Leu Thr Val Gln
            500                 505                 510

Ala Arg Gln Leu Leu Ser Gly Ile Val Gln Gln Gln Asn Asn Leu Leu
        515                 520                 525

Arg Ala Ile Glu Ala Gln Gln His Leu Leu Gln Leu Thr Val Trp Gly
    530                 535                 540

Ile Lys Gln Leu Gln Ala Arg Ile Leu Ala Val Glu Arg Tyr Leu Lys
545                 550                 555                 560

Asp Gln Gln Leu Leu Gly Ile Trp Gly Cys Ser Gly Lys Leu Ile Cys
                565                 570                 575

Thr Thr Ala Val Pro Trp Asn Ala Ser Trp Ser Asn Lys Ser Leu Glu
            580                 585                 590

Gln Ile Trp Asn His Thr Thr Trp Met Glu Trp Asp Arg Glu Ile Asn
        595                 600                 605

Asn Tyr Thr Ser Leu Ile His Ser Leu Ile Glu Glu Ser Gln Asn Gln
    610                 615                 620

Gln Glu Lys Asn Glu Gln Glu Leu Leu Glu Leu Asp Lys
625                 630                 635

<210>   5
<211>   446
<212>   PRT
<213>   Human immunodeficiency virus HIV-1

<220>
<221>   Xaa
<222>   130
<223>   Xaa is a mutant V2 loop

<400>   5
Ser Ala Thr Glu Lys Leu Trp Val Thr Val Tyr Tyr Gly Val Pro Val
1               5                   10                  15

Trp Lys Glu Ala Thr Thr Thr Leu Phe Cys Ala Ser Asp Ala Lys Ala
            20                  25                  30

Tyr Asp Thr Glu Val His Asn Val Trp Ala Thr His Ala Cys Val Pro
        35                  40                  45

Thr Asp Pro Asn Pro Gln Glu Val Val Leu Val Asn Val Thr Glu Asn
    50                  55                  60

Phe Asn Met Trp Lys Asn Asp Met Val Glu Gln Met His Glu Asp Ile
65                  70                  75                  80

Ile Ser Leu Trp Asp Gln Ser Leu Lys Pro Cys Val Lys Leu Thr Pro
                85                  90                  95

Leu Cys Val Ser Leu Lys Cys Thr Asp Leu Lys Asn Asp Thr Asn Thr
            100                 105                 110

Asn Ser Ser Ser Gly Arg Met Ile Met Glu Lys Gly Glu Ile Lys Asn
        115                 120                 125

Cys Xaa Cys Asn Thr Ser Val Ile Thr Gln Ala Cys Pro Lys Val Ser
    130                 135                 140

Phe Glu Pro Ile Pro Ile His Tyr Cys Ala Pro Ala Gly Phe Ala Ile
145                 150                 155                 160

Leu Lys Cys Asn Asn Lys Thr Phe Asn Gly Thr Gly Pro Cys Thr Asn
                165                 170                 175

Val Ser Thr Val Gln Cys Thr His Gly Ile Arg Pro Val Val Ser Thr
            180                 185                 190

Gln Leu Leu Leu Asn Gly Ser Leu Ala Glu Glu Glu Val Val Ile Arg
        195                 200                 205

Ser Val Asn Phe Thr Asp Asn Ala Lys Thr Ile Ile Val Gln Leu Asn
    210                 215                 220

Thr Ser Val Glu Ile Asn Cys Thr Arg Pro Asn Asn Asn Thr Arg Lys
225                 230                 235                 240

Arg Ile Arg Ile Gln Arg Gly Pro Gly Arg Ala Phe Val Thr Ile Gly
                245                 250                 255

Lys Ile Gly Asn Met Arg Gln Ala His Cys Asn Ile Ser Arg Ala Lys

            260                 265                 270

Trp Asn Asn Thr Leu Lys Gln Ile Ala Ser Lys Leu Arg Glu Gln Phe
        275                 280                 285

Gly Asn Asn Lys Thr Ile Ile Phe Lys Gln Ser Ser Gly Gly Asp Pro
    290                 295                 300

Glu Ile Val Thr His Ser Phe Asn Cys Gly Gly Glu Phe Phe Tyr Cys
305                 310                 315                 320

Asn Ser Thr Gln Leu Phe Asn Ser Thr Trp Phe Asn Ser Thr Trp Ser
                325                 330                 335

Thr Glu Gly Ser Asn Asn Thr Glu Gly Ser Asp Thr Ile Thr Leu Pro
            340                 345                 350

Cys Arg Ile Lys Gln Ile Ile Asn Met Trp Gln Lys Val Gly Lys Ala
        355                 360                 365

Met Tyr Ala Pro Pro Ile Ser Gly Gln Ile Arg Cys Ser Ser Asn Ile
    370                 375                 380

Thr Gly Leu Leu Leu Thr Arg Asp Gly Gly Asn Ser Asn Asn Glu Ser
385                 390                 395                 400

Glu Ile Phe Arg Pro Gly Gly Gly Asp Met Arg Asp Asn Trp Arg Ser
                405                 410                 415

Glu Leu Tyr Lys Tyr Lys Val Val Lys Ile Glu Pro Leu Gly Val Ala
            420                 425                 430

Pro Thr Lys Ala Lys Arg Arg Val Val Gln Arg Glu Lys Arg
        435                 440                 445

<210> 6
<211> 619
<212> PRT
<213> Human immunodeficiency virus HIV-1 strain SF162

<400> 6
Ser Ala Val Glu Lys Leu Trp Val Thr Val Tyr Tyr Gly Val Pro Val
1               5                   10                  15

Trp Lys Glu Ala Thr Thr Thr Leu Phe Cys Ala Ser Asp Ala Lys Ala
            20                  25                  30

Tyr Asp Thr Glu Val His Asn Val Trp Ala Thr His Ala Cys Val Pro
        35                  40                  45

Thr Asp Pro Asn Pro Gln Glu Ile Val Leu Glu Asn Val Thr Glu Asn
    50                  55                  60

Phe Asn Met Trp Lys Asn Asn Met Val Glu Gln Met His Glu Asp Ile
65                  70                  75                  80

Ile Ser Leu Trp Asp Gln Ser Leu Lys Pro Cys Val Lys Leu Thr Pro
                85                  90                  95

Leu Cys Val Thr Leu His Cys Thr Asn Leu Lys Asn Ala Thr Asn Thr
            100                 105                 110

Lys Ser Ser Asn Trp Lys Glu Met Asp Arg Gly Glu Ile Lys Asn Cys
        115                 120                 125

Ser Phe Lys Val Gly Ala Gly Lys Leu Ile Asn Cys Asn Thr Ser Val
    130                 135                 140

Ile Thr Gln Ala Cys Pro Lys Val Ser Phe Glu Pro Ile Pro Ile His
145                 150                 155                 160

Tyr Cys Ala Pro Ala Gly Phe Ala Ile Leu Lys Cys Asn Asp Lys Lys
                165                 170                 175

Phe Asn Gly Ser Gly Pro Cys Thr Asn Val Ser Thr Val Gln Cys Thr
            180                 185                 190

His Gly Ile Arg Pro Val Val Ser Thr Gln Leu Leu Leu Asn Gly Ser
        195                 200                 205

Leu Ala Glu Glu Gly Val Val Ile Arg Ser Glu Asn Phe Thr Asp Asn
    210                 215                 220

Ala Lys Thr Ile Ile Val Gln Leu Lys Glu Ser Val Glu Ile Asn Cys
225                 230                 235                 240

Thr Arg Pro Asn Asn Asn Thr Arg Lys Ser Ile Thr Ile Gly Pro Gly
                245                 250                 255

Arg Ala Phe Tyr Ala Thr Gly Asp Ile Ile Gly Asp Ile Arg Gln Ala
            260                 265                 270

His Cys Asn Ile Ser Gly Glu Lys Trp Asn Asn Thr Leu Lys Gln Ile
        275                 280                 285

Val Thr Lys Leu Gln Ala Gln Phe Gly Asn Lys Thr Ile Val Phe Lys
    290                 295                 300

Gln Ser Ser Gly Gly Asp Pro Glu Ile Val Met His Ser Phe Asn Cys
305                 310                 315                 320

Gly Gly Glu Phe Phe Tyr Cys Asn Ser Thr Gln Leu Phe Asn Ser Thr
                325                 330                 335

Trp Asn Asn Thr Ile Gly Pro Asn Asn Thr Asn Gly Thr Ile Thr Leu
            340                 345                 350

Pro Cys Arg Ile Lys Gln Ile Ile Asn Arg Trp Gln Glu Val Gly Lys
        355                 360                 365

Ala Met Tyr Ala Pro Pro Ile Arg Gly Gln Ile Arg Cys Ser Ser Asn
    370                 375                 380

Ile Thr Gly Leu Leu Leu Thr Arg Asp Gly Gly Lys Glu Ile Ser Asn
385                 390                 395                 400

Thr Thr Glu Ile Phe Arg Pro Gly Gly Gly Asp Met Arg Asp Asn Trp
                405                 410                 415

Arg Ser Glu Leu Tyr Lys Tyr Lys Val Val Lys Ile Glu Pro Leu Gly
            420                 425                 430

Val Ala Pro Thr Lys Ala Ile Ser Ser Val Val Gln Ser Glu Lys Ser
        435                 440                 445

Ala Val Thr Leu Gly Ala Met Phe Leu Gly Phe Leu Gly Ala Ala Gly
    450                 455                 460

Ser Thr Met Gly Ala Arg Ser Leu Thr Leu Thr Val Gln Ala Arg Gln
465                 470                 475                 480

Leu Leu Ser Gly Ile Val Gln Gln Gln Asn Asn Leu Leu Arg Ala Ile
                485                 490                 495

Glu Ala Gln Gln His Leu Leu Gln Leu Thr Val Trp Gly Ile Lys Gln
            500                 505                 510

Leu Gln Ala Arg Val Leu Ala Val Glu Arg Tyr Leu Lys Asp Gln Gln
        515                 520                 525

Leu Leu Gly Ile Trp Gly Cys Ser Gly Lys Leu Ile Cys Thr Thr Ala
    530                 535                 540

Val Pro Trp Asn Ala Ser Trp Ser Asn Lys Ser Leu Asp Gln Ile Trp
545                 550                 555                 560

Asn Asn Met Thr Trp Met Glu Trp Glu Arg Glu Ile Asp Asn Tyr Thr
                565                 570                 575

Asn Leu Ile Tyr Thr Leu Ile Glu Glu Ser Gln Asn Gln Gln Glu Lys
            580                 585                 590

Asn Glu Gln Glu Leu Leu Glu Leu Asp Lys Trp Ala Ser Leu Trp Asn
        595                 600                 605

Trp Phe Asp Ile Ser Lys Trp Leu Trp Tyr Ile
    610                 615